# EUROPEAN PATENT SPECIFICATION

(11) **EP 1 181 057 B1**
(45) Date of publication and mention of the grant of the patent: **01.08.2012**
(21) Application number: 00932032.6
(22) Date of filing: 03.05.2000
(51) Int. Cl.: A61K 48/00

(54) **NONINVASIVE GENETIC IMMUNIZATION, EXPRESSION PRODUCTS THEREFROM, AND USES THEREOF**
NICHT-INVASIVE GENETISCHE IMMUNISIERUNG, IHRE EXPRESSIONSPRODUKTE UND VERWENDUNGEN
IMMUNISATION GENETIQUE NON INVASIVE, PRODUITS D'EXPRESSION OBTENUS A PARTIR DE CELLE-CI ET UTILISATIONS

(30) Priority: 03.05.1999 US 132216 P; 23.03.2000 US 533149
(43) Date of publication of application: 27.02.2002
(73) Proprietor: THE UAB RESEARCH FOUNDATION, Birmingham, AL 35294-0111 (US)
(72) Inventor: TANG, De-chu, C., Birmingham, AL 35244 (US); MARKS, Donald, H., Hoover, AL 35244 (US); CURIEL, David, T., Birmingham, AL 35222 (US); SHI, Zhongkai, Birmingham, AL 35205 (US); VAN KAMPEN, Kent, R., Hoover, AL 35244 (US)
(74) Representative: Maiwald Patentanwalts GmbH
(86) International application number: PCT/US2000/012001
(87) International publication number: WO 2000/066179

(56) References cited:
- WO-A-99/08713
- US-A- 5 679 647
- US-A- 5 804 566
- US-A- 5 830 877
- SHI ET. AL.: 'DNA-based Non-Invasive Vaccination onto the Skin' VACCINE vol. 17, no. 17, 23 March 1999, pages 2136 - 2141, XP004164998
- FAN ET. AL.: 'Immunization via Hair Follicles by Topical Application of Naked DNA to Normal Skin' NATURE BIOTECHNOLOGY vol. 17, September 1999, pages 870 - 872, XP002929843
- LU ET. AL.: 'Topical Application of Viral Vectors of Epidermal Gene Transfer' J. OF INVESTIGATIVE DERMATOLOGY vol. 108, no. 5, May 1997, pages 803 - 808, XP002929844
- YOKOYAMA ET. AL.: 'DNA Immunization: Effects of Vechile and Route of Administration on the Induction of Protective Antiviral Immunity' FEMS IMMUNOLOGY AND MEDICAL MICROBIOLOGY vol. 14, 1996, pages 221 - 230, XP002910831
- NIEMIEC ET. AL.: 'Perifollicular Transgenic Expression of Human Interluekin-1 Receptor Antagonist Protein following Topical Application of Novel Liposome-Plasmid Formulations In Vivo' J. OF PHARMACEUTICAL SCIENCES vol. 86, no. 6, June 1997, pages 701 - 708, XP000690427
- WEINER, N.: 'Targeted Follicular Delivery of Macromolecules via Liposomes' INTERNATIONAL J. OF PHARMACEUTICS vol. 162, no. 1/02, 1998, pages 29 - 38, XP002913863
- Papp, Z. et al (1998) Viral Immunol. 11:79-91
- Shi, Z. et al (2001) J. Virol. 75:11474-11482

## Description

### FIELD OF THE INVENTION

The present invention relates generally to the fields of immunology and vaccine technology. The present invention also relates to techniques of non-invasive gene delivery to elicit immune responses by delivery to the nasal mucosa and uses thereof.

### BACKGROUND OF THE INVENTION

Activation of the immune system of vertebrates is an important mechanism for protecting animals against pathogens and malignant tumors. The immune system consists of many interacting components including the humoral and cellular branches. Humoral immunity involves antibodies that directly bind to antigens. Antibody molecules as the effectors of humoral immunity are secreted by B lymphocytes. Cellular immunity involves specialized cytotoxic T lymphocytes (CTLs) which recognize and kill other cells which produce non-self antigens. CTLs respond to degraded peptide fragments that appear on the surface of the target cell bound to MHC (major histocompatibility complex) class I molecules. It is understood that proteins produced within the cell are continually degraded to peptides as part of cellular metabolism. These fragments are bound to the MHC molecules and are transported to the cell surface. Thus the cellular immune system is constantly monitoring the spectra of proteins produced in all cells in the body and is poised to eliminate any cells producing non-self antigens.

Vaccination is the process of priming an animal for responding to an antigen. The antigen can be administered as a protein (classical) or as a gene which then expresses the antigen (genetic immunization). The process involves T and B lymphocytes, other types of lymphoid cells, as well as specialized antigen presenting cells (APCs) which can process the antigen and display it in a form which can activate the immune system. Current modes for the administration of genetic vaccines has focused on invasive procedures including needle injections, scarification, and gene gun-mediated penetration. Inoculation of vaccines in an invasive mode requires equipment and personnel with special medical training, and is usually associated with discomfort and potential hazards (bleeding, infection).

The efficacy of a vaccine is measured by the extent of protection against a later challenge by a tumor or a pathogen. Effective vaccines are immunogens that can induce high titer and long-lasting protective immunity for targeted intervention against diseases after a minimum number of inoculations. For example, genetic immunization is an approach to elicit immune responses against specific proteins by expressing genes encoding the proteins in an animal's own cells. The substantial antigen amplification and immune stimulation resulting from prolonged antigen presentation *in vivo* can induce a solid immunity against the antigen. Genetic immunization simplifies the vaccination protocol to produce immune responses against particular proteins because the often difficult steps of protein purification and combination with adjuvant, both routinely required for vaccine development, are eliminated. Since genetic immunization does not require the isolation of proteins, it is especially valuable for proteins that may lose conformational epitopes when purified biochemically. Genetic vaccines may also be delivered in combination without eliciting interference or affecting efficacy (Tang et al., 1992; Barry et al., 1995), which may simplify the vaccination scheme against multiple antigens.

While topically-applied protein-based vaccines have been studied, their usefulness may be limited. Although topical application of protein-cased vaccines in conjunction with cholera toxin may also immunize animals in a non-invasive mode (Glenn et al., 1998), skin-targeted non-invasive genetic vaccines as disclosed in the present invention activate the immune system via a different mechanism than protein-based vaccines. Further, the efficacy of genetic vaccines is in general superior to that of protein vaccines due to the *de novo* synthesis of antigens similar to natural infections (McDonnell and Askari, 1996). Although U.S. Pat. No. 3,837,340 relates to a method for vaccinating animals by contacting skin with dried viruses, the viruses that are employed therein are not genetic vectors capable of expressing transgenes or heterologous or exogenous nucleic acid molecules. In addition, the immunogen may be protein in the viral coat, instead of protein produced from expression of viral genes in the animals' own cells, e.g., any immunological response induced by U.S. Patent No. 3,837,340 can be akin to that which is induced by topical application of protein-based vaccines which are non-analogous to the present invention and ergo U.S. Patent No. 3,837,340 is non-analogous to the present invention.

The prior art of vaccination usually requires equipment, e.g., syringe needles or a gene gun, and special skill for the administration of vaccines. There is a great need and desire in the art for the inoculation of vaccines by personnel without medical training and equipment.

WO 99/08713 pubished on February 25, 1995 discloses a method of inducing an immune response in a non-invasive mode, comprising the step of contacting skin of an individual in need of such treatment topically by applying to said skin an immunologically effective concentration of a genetic vector encoding a gene of interest.

Shi et al. (entitled "DNA-based non-invasive vaccination onto the skin", Vaccine Vol. 17, no. 17, 23 March 1999, pages 2136-2141) discloses that non-invasive vaccination onto the skin (NIVS) could improve vaccination programs because the procedure requires no specially trained personnel and may eliminate many problems associated with needle injections. Further, Shi et al. discusses that the obtained results may provide a proof of principle that NIVS may appear as a novel method for the administration of DNA-based vaccines.

Papp et al. (entitled "Induction of immunity in the respiratory tract and protection from bovine herpesvirus type I infection by different routes of immunization with recombinatn adenovirus", Viral Immunology, Vol. 11., no. 2, 1998, pages 79-91) discloses an investigation of the capability of different routes of immunization with replication-competent recombinant adenovirus to induce antigen-specific antibody responses.

### OBJECTS AND SUMMARY OF THE INVENTION

Non-invasive vaccination onto the skin (NIVS) can improve vaccination schemes because skin is an immunocompetent tissue and this non-invasive procedure requires no specially trained personnel. Skin-targeted non-invasive gene delivery can achieve localized transgene expression in the skin and the elicitation of immune responses (Tang et al., 1997) and the mechanism for these responses is different than that from topical application of protein-based vaccines in conjunction with cholera toxin (Glenn et al., 1998). These results indicate that vector-based NIVS is a novel and efficient method for the delivery of vaccines. The simple, effective, economical and painless immunization protocol dislcosed in present invention should make vaccination less dependent upon medical resources and, therefore, increase the annual utilization rate of vaccinations.

The present invention provides the use of an adenoviral vector as defined in independent claim 1. The invention also provides an adenoviral vector for use as defined in independent claim 1.

, namely a non-invasoive vaccine for mucosal administration for use as defined in independent claim 21.

The present invention also encompasses a kit. Such a kit comprises the vector and a pharmaceutically acceptable or suitable carrier or diluent and an optional delivery device, each in its own packaging; the packaging may be included in a unitary container or the packaging may each be in separate containers or as defined in independent claim 29. each may be its own separate container; the kit can optionally include instructions for admixture of the ingredients and/or administration of the composition.

Pour-on and spot-on formulations are described in U.S. Patents Nos. 6,010,710 and 5,475,005. A roll-on device is also described in U.S. Patent No. 5,897,267. Moreover, a skilled artisan also knows make shampoo formulation as well as devices to apply the formulation to an animal.

Preferred embodiments are referred to in the dependent claims.

It is noted that in this disclosure, terms such as "comprises", "comprised", "comprising" and the like can have the meaning attributed to it in U.S. Patent law; e.g., they can mean "includes", "included", "including" and the like.

These and other embodiments are disclosed or are obvious from and encompassed by, the following Detailed Description.

### BRIEF DESCRIPTION OF FIGURES

The following Detailed Description, given by way of example, but not intended to limit the invention to specific embodiments described, may be understood in conjunction with the accompanying Figures, incorporated herein by reference, in which:
Figure 1 shows the transgene expression from adenovirus recombinants in the skin by topical application of the vectors;
Figures 2a and 2b show the characterization of potential target cells that can be transduced by topically-applied adenovirus recombinants;
Figures 3a and 3b show the detection of specific antibodies in the sera of mice immunized by adenovirus-mediated NIVS;
Figure 4 shows the percent survival of control versus immunized mice that were challenged by a lethal dose of tumor cells;
Figure 5 shows the characterization of tumor-infiltrating T lymphocytes;
Figure 6 shows the characterization of tumor-infiltrating CTLs;
Figure 7 shows the western blot analysis of antibodies to the human CEA protein in mice immunized by topical application of vaccine bandages;
Figure 8a shows the detection of specific antibodies in the serum of a mouse immunized by DNA/adenovirus-mediated NIVS;
Figure 8b shows the detection of specific antibodies in the serum of a mouse immunized by DNA/liposome-mediated NIVS;
Figure 9 shows the co-expression of DNA-encoded and adenovirus-encoded transgenes in target cells;
Figure 10 shows relative transgene expression from topically-applied adenovirus recombinants, DNA/adenovirus complexes, and DNA/liposome complexes;

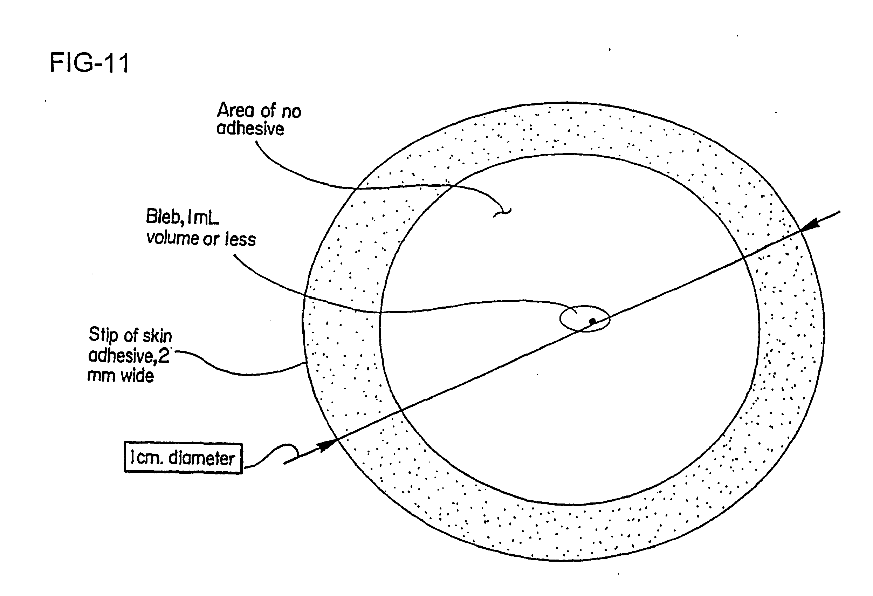

Figure 11 shows a device for the administration of skin-targeted non-invasive vaccines.
Figure 12 shows anti-influenza antibodies generated by skin-targeted noninvasive vaccines in mice;
Figure 13 shows protection of mice from death following virus challenge.
Figure 14 shows ELISA antibodies generated in a pigtail macaque by a skin patch containing an adenovirus vector encoding influenza HA;
Figure 15 shows relocation of antigen spots in skin after topical application of an adenovirus vector;
Figure 16 shows amplification of foreign DNA in various tissues after localized gene delivery in a noninvasive mode;
Figure 17 shows that a depilatory agent such as NAIR is not essential for NIVS;
Figure 18 shows protection from death following *Clostridium tetani* challenge by topical application or intranasal inoculation of an adenovirus-based tetanus vaccine.

### DETAILED DESCRIPTION

Inoculation of vaccines in an invasive mode may be unnecessary (Tang et al., 1997; Glenn et al., 1998). Since the skin interfaces directly with the external environment and is in constant contact with potential pathogens, the immune system must constantly keep a mobilized biological army along the skin border for warding off potential infections. As a consequence, the outer layer of skin is essentially an immunocompetent tissue. Immunologic components present in the skin for the elicitation of both humoral and cytotoxic cellular immune responses include epidermal Langerhans cells (which are MHC class II-positive antigen-presenting cells), keratinocytes, and both CD4⁺ and CD8⁺ T lymphocytes. These components make the skin an ideal site for administration of vaccine. The large accessible area of skin and its durability are other advantages for applying vaccines to this tissue. Expression of a small number of antigens in the outer layer of skin without physical penetration may thus elicit a potent immune response by alarming the immune surveillance mechanism.

It is herein demonstrated that genetic vaccines can be inoculated in a novel way as skin-targeted non-invasive vaccine, or immunogenic, or immunological or therapeutic compositions. The combination of genetic vaccines with a non-invasive delivery mode results in a new class of "democratic" vaccine, or immunogenic, or immunological or therapeutic compositions that may require little or no special skill and equipment for administration. Thus, one can administer such compositions to the skin of himself or herself (and, this administration can advantageously be under the direction of a medical practitioner, e.g., to ensure that dosage is proper) or to the skin of an animal (e.g., advantageously a shaved area of skin if the animal is a mammal, although as demonstrated herein, hair removal is not necessary, and more advantageously at a region where the animal will not remove the administration by rubbing, grooming or other activity); and, the present invention thus discloses advantages in the administration of vaccine, or immunogenic, or immunological, or therapeutic compositions comprising a vector that expresses a gene product, especially with respect to administering such compositions to newborns, young animals, animals generally, children and the like, to whom invasive, e.g., needle, administration may be somewhat difficult or inconvenient or painful.

As used herein, a vector is a tool that allows or facilitates the transfer of an entity from one environment to another. By way of example, some vectors used in recombinant DNA techniques allow entities, such as a segment of DNA (such as a heterologous DNA segment, such as a heterologous cDNA segment), to be transferred into a target cell.

As used herein, "AdCMV-tetC:IM" represents an adenovirus vector encoding the *Clotridium tetani* toxin C-fragment; "pCMV-tetC" represents a plasmid expression vector encoding the *Clotridium tetani* toxin C-fragment.

Reference is made to U.S. Patent No. 5,990,091 issued November 23, 1999, Einat et al. or Quark Biotech, Inc., WO 99/60164, published November 25, 1999 from PCT/US99/11066, filed May 14, 1999, Fischer or Rhone Merieux, Inc., WO98/00166, published January 8, 1998 from PCT/US97/11486, filed June 30, 1997 (claiming priority from U.S. applications Serial Nos. 08/675,556 and 08/675,566), van Ginkel et al., J. Immunol 159(2):685-93 (1997) ("Adenoviral gene delivery elicits distinct pulmonary-associated T helper cell responses to the vector and to its transgene"), Osterhaus et al., Immunobiology 184(2-3):180-92 (1992) ("Vaccination against acute respiratory virus infections and measles in man"), Briles et al. or UAB, WO 99/53940, published October 28, 1999 from PCT/US99/08895, filed April 23, 1999, and Briles et al. or UAB, U.S. Patent No. 6,042,838, issued March 28, 2000, and Briles et al. or UAB U.S. Patent No. 6,004,802, for information concerning expressed gene products, antibodies and uses thereof, vectors for *in vivo* and *in vitro* expression of exogenous nucleic acid molecules, promoters for driving expression or for operatively linking to nucleic acid molecules to be expressed, method and documents for producing such vectors, compositions comprising such vectors or nucleic acid molecules or antibodies, dosages, and modes and/or routes of administration (including compositions for mucosal, nasal, oral, oral cavity, buccal, perlingual administration). And thus, U.S. Patent No. 5,990,091 issued November 23, 1999, Einat et al. or Quark Biotech, Inc., WO 99/60164, published November 25, 1999 from PCT/US99/11066, filed May 14, 1999, Fischer or Rhone Merieux, Inc., WO98/00166, published January 8, 1998 from PCT/US97/11486, filed June 30, 1997 (claiming priority from U.S. applications Serial Nos. 08/675,556 and 08/675,566), van Ginkel et al., J. Immunol 159(2):685-93 (1997) ("Adenoviral gene delivery elicits distinct pulmonary-associated T helper cell responses to the vector and to its transgene"), Osterhaus et al., Immunobiology 184(2-3):180-92 (1992) ("Vaccination against acute respiratory virus infections and measles in man"), Briles et al. or UAB, WO 99/53940, published October 28, 1999 from PCT/US99/08895, filed April 23, 1999, and Briles et al. or UAB, U.S. Patent No. 6,042,838, issued March 28, 2000 and Briles et al. or UAB, U.S. Patent No. 6,004,802, and all documents cited or referenced therein and all documents cited or referenced in documents referenced or cited in each of U.S. Patent No. 5,990,091 issued November 23, 1999, Einat et al. or Quark Biotech, Inc., WO 99/60164, published November 25, 1999 from PCT/US99/11066, filed May 14, 1999, Fischer or Rhone Merieux, Inc., WO98/00166, published January 8, 1998 from PCT/US97/11486, filed June 30, 1997 (claiming priority from U.S. applications Serial Nos. 08/675,556 and 08/675,566), van Ginkel et al., J. Immunol 159(2):685-93 (1997) ("Adenoviral gene delivery elicits distinct pulmonary-associated T helper cell responses to the vector and to its transgene"), Osterhaus et al., Immunobiology 184(2-3):180-92 (1992) ("Vaccination against acute respiratory virus infections and measles in man"), Briles et al. or UAB, WO 99/53940, published October 28, 1999 from PCT/US99/08895, filed April 23, 1999, and Briles et al. or UAB, U.S. Patent No. 6,042,838, issued March 28, 2000, and Briles et al. or UAB U.S. Patent No. 6,004,802 Information in U.S. Patent No. 5,990,091 issued November 23, 1999, WO 99/60164, WO98/00166, van Ginkel et al., J. Immunol 159(2):685-93 (1997), Osterhaus et al., Immunobiology 184(2-3):180-92 (1992), WO 99/53940 and U.S. Patents Nos. 6,042,838 and 6,004,802, can be relied upon for the practice of this disclosure (e.g., expressed products, antibodies and uses thereof, vectors for *in vivo* and *in vitro* expression of exogenous nucleic acid molecules, exogenous nucleic acid molecules encoding epitopes of interest or antigens or therapeutics and the like, promoters, compositions comprising such vectors or nucleic acid molecules or expressed products or antibodies, dosages, *inter alia).* It is noted that immunological products and/or antibodies and/or expressed products obtained in accordance with this invention can be expressed *in vitro* and used in a manner in which such immunological and/or expressed products and/or antibodies are typically used, and that cells that express such immunological and/or expressed products and/or antibodies can be employed in *in vitro* and/or *ex vivo* applications, e.g., such uses and applications can include diagnostics, assays, *ex vivo* therapy (e.g., wherein cells that express the gene product and/or immunological response are expanded *in vitro* and reintroduced into the host or animal), etc., *see* U.S. Patent No. 5,990,091, WO 99/60164, WO 98/00166, WO 99/53940, and U.S. Patents Nos. 6,042,838, and 6,004,802, and documents cited therein and documents cited or referenced in such documents. Further, expressed antibodies or gene products that are isolated from herein methods, or that are isolated from cells expanded *in vitro* following herein administration methods, can be administered in compositions, akin to the administration of subunit epitopes or antigens or therapeutics or antibodies to induce immunity, stimulate a therapeutic response and/or stimulate passive immunity. The quantity to be administered will vary for the patient (host) and condition being treated and will vary from one or a few to a few hundred or thousand micrograms, e.g., 1 µg to 1 mg, from about 100 ng/kg of body weight to 100 mg/kg of body weight per day and preferably will be from 10 pg/kg to 10 mg/kg per day. A vector can be non-invasively administered to a patient or host in an amount to achieve the amounts stated for gene product (e.g., epitope, antigen, therapeutic, and/or antibody) compositions. Of course, the invention envisages dosages below and above those exemplified herein, and for any composition to be administered to an animal or human, including the components thereof, and for any particular method of administration, it is preferred to determine therefor: toxicity, such as by determining the lethal dose (LD) and LD₅₀ in a suitable animal model e.g., rodent such as mouse; and, the dosage of the composition(s), concentration of components therein and timing of administering the composition(s), which elicit a suitable response, such as by titrations of sera and analysis thereof, e.g., by ELISA and/or seroneutralization analysis. Such determinations do not require undue experimentation from the knowledge of the skilled artisan, this disclosure and the documents cited herein. And, the invention also comprehends sequential administration of inventive compositions or sequential performance of herein methods, e.g., periodic administration of inventive compositions such as in the course of therapy or treatment for a condition and/or booster administration of immunological compositions and/or in prime-boost regimens; and, the time and manner for sequential administrations can be ascertained without undue experimentation. Further, the invention discloses compositions and methods for making and using vectors, including methods for producing gene products and/or immunological products and/or antibodies *in vivo* and/or in *vitro* and/or *ex vivo* (e.g., the latter two being, for instance, after isolation therefrom from cells from a host that has had a non-invasive administration according to the invention, e.g., after optional expansion of such cells), and uses for such gene and/or immunological products and/or antibodies, including in diagnostics, assays, therapies, treatments, and the like. Vector compositions are formulated by admixing the vector with a suitable carrier or diluent; and, gene product and/or immunological product and/or antibody compositions are likewise formulated by admixing the gene and/or immunological product and/or antibody with a suitable carrier or diluent; *see, e.g.,* U.S. Patent No. 5,990,091, WO 99/60164, WO 98/00166, WO 99/53940, and U.S. Patents Nos. 6,042,838 and 6,004,802, documents cited therein, and other documents cited herein, and other teachings herein (for instance, with respect to carriers, diluents and the like).

If nasal or respiratory (mucosal) administration is desired, compositions may be in a form and dispensed by a squeeze spray dispenser, pump dispenser or aerosol dispenser. Such dispensers may also be employed to deliver the composition to oral or oral cavity (e.g., buccal or perlingual) mucosa. Aerosols are usually under pressure by means of a hydrocarbon. Pump dispensers can preferably dispense a metered dose or, a dose having a particular particle size.

Compositions disclosed in the invention can contain pharmaceutically acceptable flavors and/or colors for rendering them more appealing, especially if they are administered orally (or buccally or perlingually); and, such compositions can be in the form of tablets or capsules that dissolve in the mouth or which are bitten to release a liquid for absorption buccally or perlingually (akin to oral, perlingual or buccal medicaments for angina such as nitroglycerin or nifedimen). The viscous compositions may be in the form of gels, lotions, ointments, creams and the like (e.g., for topical and/or mucosal and/or nasal and/or oral and/or oral cavity and/or perlingual and/or buccal administration), and will typically contain a sufficient amount of a thickening agent so that the viscosity is from about 2500 to 6500 cps, although more viscous compositions, even up to 10,000 cps may be employed. Viscous compositions have a viscosity preferably of 2500 to 5000 cps, since above that range they become more difficult to administer. However, above that range, the compositions can approach solid or gelatin forms which are then easily administered as a swallowed pill for oral ingestion and/or a pill or capsule or tablet for holding in the mouth, e.g., for buccal or perlingual administration.

Liquid preparations are normally easier to prepare than gels, other viscous compositions, and solid compositions. Additionally, liquid compositions are somewhat more convenient to administer, especially by injection or orally or buccally or perlinually, to animals, children, particularly small children, and others who may have difficulty swallowing a pill, tablet, capsule or the like, or in multi-dose situations. Viscous compositions, on the other hand, can be formulated within the appropriate viscosity range to provide longer contact periods with mucosa, such as the lining of the stomach or nasal mucosa or for perlingual or buccal or oral cavity absorption.

Obviously, the choice of suitable carriers and other additives will depend on the exact route of administration and the nature of the particular dosage form, e.g., liquid dosage form (e.g., whether the composition is to be formulated into a solution, a suspension, gel or another liquid form), or solid dosage form (e.g., whether the composition is to be formulated into a pill, tablet, capsule, caplet, time release form or liquid-filled form).

Solutions, suspensions and gels, normally contain a major amount of water (preferably purified water) in addition to the antigen, lipoprotein and optional adjuvant. Minor amounts of other ingredients such as pH adjusters (e.g., a base such as NaOH), emulsifiers or dispersing agents, buffering agents, preservatives, wetting agents, jelling agents, (e.g., methylcellulose), colors and/or flavors may also be present. The compositions can be isotonic, i.e., it can have the same osmotic pressure as blood and lacrimal fluid.

The desired isotonicity of the compositions disclosed in the invention may be accomplished using sodium chloride, or other pharmaceutically acceptable agents such as dextrose, boric acid, sodium tartrate, propylene glycol or other inorganic or organic solutes. Sodium chloride is preferred particularly for buffers containing sodium ions.

Viscosity of the compositions may be maintained at the selected level using a pharmaceutically acceptable thickening agent. Methylcellulose is preferred because it is readily and economically available and is easy to work with. Other suitable thickening agents include, for example, xanthan gum, carboxymethyl cellulose, hydroxypropyl cellulose, carbomer, and the like. The preferred concentration of the thickener will depend upon the agent selected. The important point is to use an amount which will achieve the selected viscosity. Viscous compositions are normally prepared from solutions by the addition of such thickening agents.

A pharmaceutically acceptable preservative can be employed to increase the shelf-life of the compositions. Benzyl alcohol may be suitable, although a variety of preservatives including, for example, parabens, thimerosal, chlorobutanol, or benzalkonium chloride may also be employed. A suitable concentration of the preservative will be from 0.02% to 2% based on the total weight although there may be appreciable variation depending upon the agent selected.

Those skilled in the art will recognize that the components of the compositions must be selected to be chemically inert with respect to the vector or antigen or epitope of interest and optional adjuvant or other active or immunity-enhancing ingredients. This will present no problem to those skilled in chemical and pharmaceutical principles, or problems can be readily avoided by reference to standard texts or by simple experiments (not involving undue experimentation), from this disclosure and the documents cited herein.

The immunologically effective compositions disclosed in the invention are prepared by mixing the ingredients following generally accepted procedures. For example the selected components may be simply mixed in a blender, or other standard device to produce a concentrated mixture which may then be adjusted to the final concentration and viscosity by the addition of water or thickening agent and possibly a buffer to control pH or an additional solute to control tonicity. Generally the pH may be from about 3 to 7.5. Compositions can be administered in dosages and by techniques well known to those skilled in the medical and veterinary arts taking into consideration such factors as the age, sex, weight, and condition of the particular patient or animal, and the composition form used for administration (e.g., solid vs. liquid). Dosages for humans or other mammals can be determined without undue experimentation by the skilled artisan, from this disclosure, the documents cited herein, the Examples below and from the applications, patents and other documents cited herein and documents cited or referenced in documents cited herein, all of which are incorporated herein by reference.

Suitable regimes for initial administration and booster doses or for sequential administrations also are variable, and may include an initial administration followed by subsequent administrations; but nonetheless, may be ascertained by the skilled artisan, from this disclosure, the documents cited and incorporated by reference herein, including applications and patents cited herein and documents referenced or cited in herein cited documents, all of which are hereby incorporated herein by reference, as well as the Examples below. The compositions can be administered alone, or can be co-administered or sequentially administered with other compositions of the invention or with other prophylactic or therapeutic compositions.

The vector expresses a gene which z encodes influenza hemagglutinin and/or influenza nuclear protein.

In an embodiment of the invention, the immune response in the animal is induced by genetic vectors expressing genes encoding antigens of interest in the animal's cells, namely influenza hemagglutinin and/or influenza nuclear protein. The genetic vector is used as a prophylactic vaccine or a therapeutic vaccine. In another embodiment of the invention, the genetic vector comprises genetic vectors capable of expressing an antigen of interest in the animal's cells. In a further embodiment of the method, the animal is a vertebrate.

With respect to exogenous DNA for expression in a vector (e.g., encoding an epitiope of interest and/or an antigen and/or a therapeutic) and documents providing such exogenous DNA, as well as with respect to the expression of transcription and/or translation factors for enhancing expression of nucleic acid molecules, and as to terms such as "epitope of interest", "therapeutic", "immune response", "immunological response", "protective immune response", "immunological composition", "immunogenic composition", and "vaccine composition", *inter alia,* reference is made to U.S. Patent No. 5,990,091 issued November 23, 1999, and WO 98/00166 and WO 99/60164, and the documents cited therein and the documents of record in the prosecution of that patent and those PCT applications. Thus, U.S. Patent No. 5,990,091 and WO 98/00166 and WO 99/60164 and documents cited therein and documents or record in the prosecution of that patent and those PCT applications, and other documents cited herein or otherwise incorporated herein by reference, can be consulted in the practice of this invention; and, all exogenous nucleic acid molecules, promoters, and vectors cited therein can be used in the practice of this invention. In this regard, mention is also made of U.S. Patents Nos. 6,004,777, 5,997,878, 5,989,561, 5,976,552, 5,972,597, 5,858,368, 5,863,542, 5,833,975, 5,863,542, 5,843,456, 5,766,598, 5,766,597, 5,762,939, 5,756,102, 5,756,101, 5,494,807, 6,042,838, 6,004,802 and WO 99/53940.

In another embodiment of the invention, the animal is advantageously a vertebrate such as a mammal, bird, reptile, amphibian or fish; more advantageously a human, or a companion or domesticated or food-producing or feed-producing or livestock or game or racing or sport animal such as a cow, a dog, a cat, a goat, a sheep or a pig or a horse, or even fowl such as turkey, ducks or chicken. In an especially advantageous another embodiment of the invention, the vertebrate is a human.

In another embodiment of the invention, the immune response is against influenza A. In another embodiment of the invention, the immune response against influenza A is induced by the genetic vector expressing a gene encoding an influenza hemagglutinin and/or an influenza nuclear protein or a fragment thereof in the animal's cells. In the present invention, the genetic vector is an adenovirus defective in its E1 and E3 regions. In another embodiment of the invention, the DNA is in plasmid form.

In another embodiment of the invention, the vector has all viral genes deleted.

In another embodiment of the present invention, the vector further contains a gene selected from the group consisting of co-stimulatory genes and cytokine genes. In this method the gene is selected from the group consisting of a GM-CSF gene, a B7-1 gene, a B7-2 gene, an interleukin-2 gene, an interleukin-12 gene and interferon genes.

Embodiments of the invention that employ adenovirus recombinants include E1-defective and E3-defective adenovirus vectors, or the "gutless" adenovirus vector in which all viral genes are deleted. The E1 mutation raises the safety margin of the vector because E1-defective adenovirus mutants are replication incompetent in non-permissive cells. The E3 mutation enhances the immunogenicity of the antigen by disrupting the mechanism whereby adenovirus down-regulates MHC class I molecules. The "gutless" adenovirus vector is the latest model in the adenovirus vector family. Its replication requires a helper virus and a special human 293 cell line expressing both E1a and Cre, a condition that does not exist in natural environment; the vector is deprived of all viral genes, thus the vector as a vaccine carrier is non-immunogenic and may be inoculated for multiple times for re-vaccination. The "gutless" adenovirus vector also contains 36 kb space for accommodating transgenes, thus allowing co-delivery of a large number of antigen genes into cells. Specific sequence motifs such as the RGD motif may be inserted into the H-I loop of an adenovirus vector to enhance its infectivity. An adenovirus recombinant is constructed by cloning specific transgenes or fragments of transgenes into any of the adenovirus vectors such as those described above. The adenovirus recombinant is used to transduce epidermal cells of a vertebrate in a non-invasive mode for use as an immunizing agent.

Embodiments that use DNA/adenovirus complexes can have the plasmid DNA complexed with adenovirus vectors utilizing a suitable agent therefor, such as either PEI (polyethylenimine) or polylysine. The adenovirus vector within the complex may be either "live" or "killed" by UV or gamma irradiation. The irradiation-inactivated adenovirus vector as a receptor-binding ligand and an endosomolysis agent for facilitating DNA-mediated transfection (Cotten et al., 1992) may raise the safety margin of the vaccine carrier. The DNA/adenovirus complex is used to transfect epidermal cells of a vertebrate in a non-invasive mode for use as an immunizing agent.

Embodiments that use DNA/liposome complexes can have materials for forming liposomes, and DNA/liposome complexes be made from these materials. The DNA/liposome complex is used to transfect epidermal cells ot a vertebrate in a non-invasive mode for use as an immunizing agent.

Genetic vectors disclosed in the invention can also code for immunomodulatory molecules which can act as an adjuvant to provoke a humoral and/or cellular immune response. Such molecules include cytokines, co-stimulatory molecules, or any molecules that may change the course of an immune response. One can conceive of ways in which this technology can be modified to enhance still further the immunogenicity of antigens.

Dosage forms for the topical administration of the genetic vector and gene of interest of this invention can include liquids, ointments, powders, and sprays. The active component can be admixed under sterile conditions with a physiologically acceptable carrier and any preservatives, buffers, propellants, or absorption enhancers as may be required or desired. Reference is made to documents cited herein, e.g., U.S. Patents Nos. 5,990,091, 6,042,838, and 6,004,802, and WO 98/00166 and WO 99/60164, and WO 99/53940, and documents cited therein for methods for constructing vectors, as well as for compositions for topical application, e.g., viscous compositions that can be creams or ointments, as well as compositions for nasal administration.

In terms of the terminology used herein, an immunologically effective amount is an amount or concentration of the genetic vector encoding the gene of interest, that, when administered to an animal, produces an immune response to the gene product of interest.

Various epitopes, antigens or therapeutics may be delivered topically by expression thereof at different concentrations. Generally, useful amounts for adenovirus vectors are at least approximately 100 pfu and for plasmid DNA at least approximately 1 ng of DNA. Other amounts can be ascertained from this disclosure and the knowledge in the art, including documents cited and incorporated herein by reference, without undue experimentation.

The methods disclosed in the invention can be appropriately applied to prevent diseases as prophylactic vaccination or treat diseases as therapeutic vaccination.

The vaccines of the present invention can be administered to an animal either alone or as part of an immunological composition.

Beyond the human vaccines described, the method of the invention can be used to immunize animal stocks. The term animal means all animals including humans. Examples of animals include humans, cows, dogs, cats, goats, sheep, horses, pigs, turkey, ducks and chicken, etc. Since the immune systems of all vertebrates operate similarly, the applications described can be implemented in all vertebrate systems.

The following examples are given for the purpose of illustrating various embodiments of the invention and are not meant to limit the present invention in any fashion.

### EXAMPLES

### Protocols

### Mice and cell cultures

Inbred mice were maintained at the University of Alabama at Birmingham. Cells were cultured in RPMI 1640 or DMEM media containing 2% fetal bovine serum and 6% calf serum.

### Topical application of genetic vectors

Mice were anesthetized and hair and cornified epithelium covering a restricted area of abdominal or neck skin were removed by a brush or a depilatory (e.g., NAIR). Genetic vectors were pipetted onto the preshaved skin and kept in contact with naked skin for varying amounts of time (e.g., 1 hour to 18 hours). Vectors may be pipetted directly onto naked skin, or into a cylinder that is glued onto the skin.

### Preparation of adenovirus vectors

High titer adenovirus stocks were prepared from human 293 cells infected with specific adenovirus recombinants. Lysates were subjected to ultracentrifugation through a cesium chloride gradient. Viral bands were extracted and dialyzed against 10 mM Tris (pH 7.5)/135 mM NaCl/5 mM KCl/l mM MgCl₂. Purified viruses were filter sterilized with glycerol added to 10%, and stored in aliquots at -80°C. Titer for adenovirus stocks was determined by plaque assay.

### Luciferase assay

The amount of luciferase in the skin was determined as previously described (Tang, 1994). Briefly, a piece of excised skin was homogenized with a Kontes glass tissue grinder in lysis buffer. After removing tissue debris by centrifugation, luciferase activity in the skin extract was determined with a luminometer by measurement of integrated light emission in the presence of excess ATP and luciferin.

### β-Galactosidase assay

A piece of excised skin was quickly frozen in Tissue-Tek O.C.T. compound (Miles Laboratories Inc.) in liquid nitrogen and stored at -80°C until use. The frozen tissue was cross sectioned at 4 µm, fixed in 4% paraformaldehyde, and stained for β-galactosidase activity by incubation in X-gal staining solution as previously described (Tang et al., 1994). Sections were counterstained with haematoxylin and eosin.

### Preparation of DNA/adenovirus complexes

DNA/adenovirus complexes were prepared by mixing 100 µg plasmid DNA with 1 x 10¹¹ particles of adenovirus in the presence of the condensing agent polylysine for each application. The titer of adenovirus was determined by absorbance.

### Preparation of DNA/liposome complexes

DNA/liposome complexes were prepared by mixing 100 µg plasmid DNA with 100 µg DOTAP/DOPE (1:1; Avanti) for each application. Plasmids were prepared using Qiagen Plasmid Maxi Kits.

### Western blot analysis

Sera from tail bleeds were diluted 1:250 to 1:500 and reacted with purified proteins that had been separated in a SDS-polyacrylamide gel and transferred to an Immobilon-P membrane (Millipore). Reaction was visualized using the ECL kit (Amersham).

### Reference EXAMPLE 1

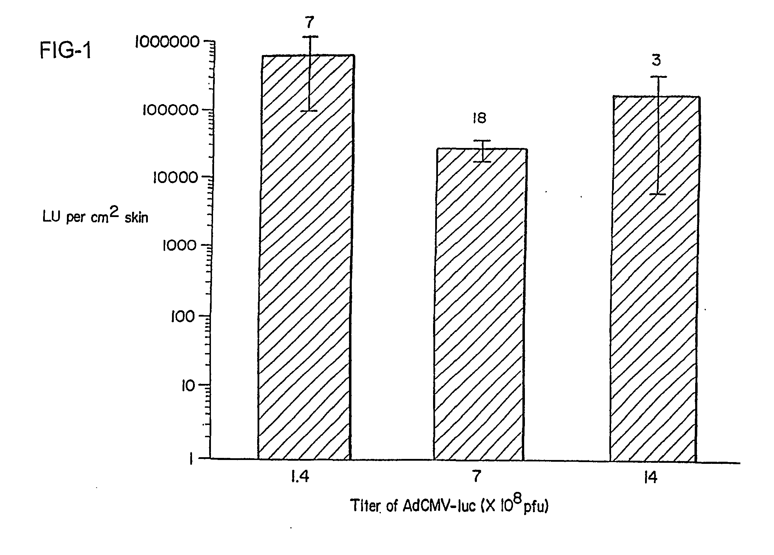

The present invention demonstrates that antigen genes can be delivered into the skin of mice in a simplified manner by skin-targeted non-invasive delivery of a genetic vector without using sophisticated equipment. Figure 1 shows that substantial amounts of luciferase enzyme was produced after delivery of limited amounts of AdCMV-luc (an adenovirus vector encoding the firefly luciferase) (Tang et al., 1994) onto the skin. Ad, adenovirus; pfu, plaque-forming units; LU, light units. Results are the mean log[LU per cm² skin] ± SE (n is shown on top of each column). Mice mock-applied or coated with an adenovirus vector that did not encode luciferase produced no detectable luciferase activity in the skin. The level of transgene expression from the adenovirus vector in the skin did not appear to correlate with the titer of the virus. It is possible that only a small number of cells can be transduced by the virus in a restricted subset of skin, and 10⁸ plaque-forming units (pfu) of adenovirus recombinants may have saturated the target cells. This variability could also be due, in part, to variations of individual mice. In addition, some of the variability probably arose from the procedure for removing cornified epithelium which had not been standardized (Johnston and Tang, 1994). The amount of antigen produced may potentially be amplified by applying more vectors onto a larger area.

### Reference EXAMPLE 2

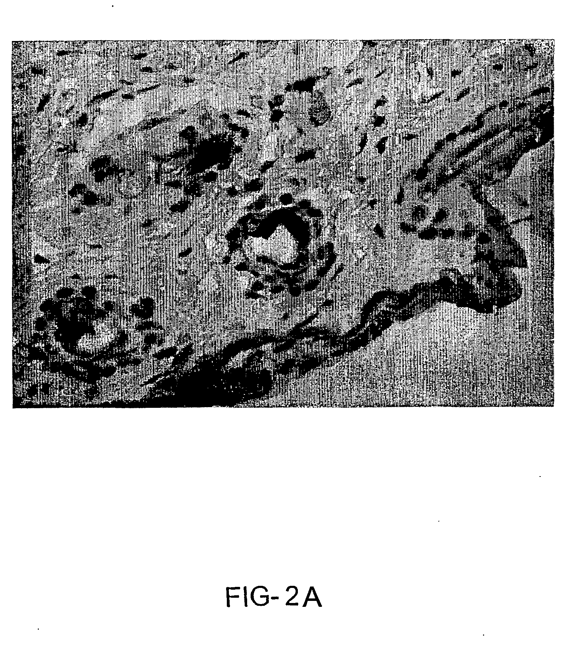

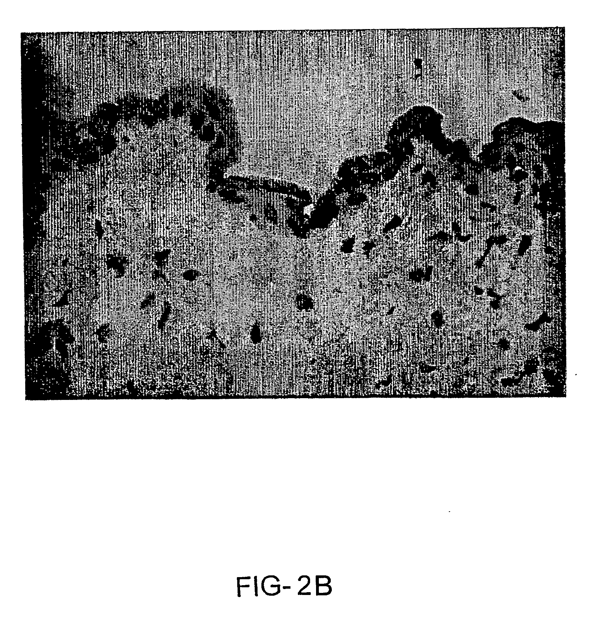

The principal target cells for non-invasive vaccination onto the skin appeared to be hair matrix cells within hair follicles (Figure 2a) and keratinocytes within the outermost layer of epidermis (Figure 2b) as shown by staining frozen sections with X-gal substrates after skin-targeted non-invasive delivery of an adenovirus vector encoding the *E*. *coli* β-galactosidase gene (AdCMV-βgal) (Tang et al., 1994). No physical abrasions were found in the skin tissue subjected to the treatment, and there was no inflammation induced. The skin tissue subjected to non-invasive gene delivery was excised from animals 1 day after pipetting 10⁸ pfu of AdCMV-βgal onto the skin, cross sectioned, fixed, and stained with X-gal substrates as described (Tang et al., 1994). Figure 2a shows the adenovirus-transduced hair matrix cells within a hair follicle, x 150. Figure 2b shows the adenovirus-transduced keratinocytes within the outermost layer of epidermis, x150. No blue cells were found in control animals that were either mock-applied or coated with AdCMV-luc.

### Reference EXAMPLE 3

### Elicitation of humoral immune responses by adenovirus-mediated NIVS

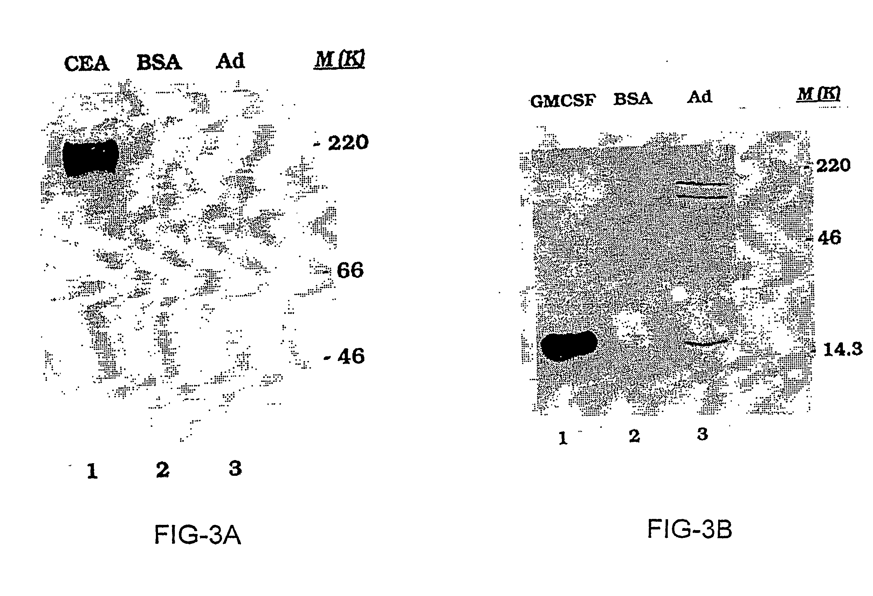

NIVS is a novel method for vaccinating animals. To demonstrate that the procedure can elicit a specific immune response against the antigen encoded by the vector, AdCMV-hcea (an adenovirus vector encoding the human carcinoembryonic antigen (CEA)) was pipetted onto the skin of the C57BL/6 strain mice. Serum from a vaccinated mouse a month after skin-targeted non-invasive delivery of 10⁸ pfu AdCMV-hcea was diluted 1:500 and reacted with purified human CEA protein (provided by T. Strong) and adenoviral proteins that had been separated in a 5% SDS-polyacrylamide gel, and transferred to Immobilon-P membranes (Millipore). Referring to Figure 3a, lane 1, 0.5 µg of human CEA; lane 2, 0.5 µg of BSA; lane 3, 10⁷ pfu of adenovirus. Figure 3a shows that the test sera from a vaccinated animal reacted in western blots with purified human CEA protein, but not with bovine serum albumin (BSA), which supports the conclusion that specific antibodies have been produced against exogenous proteins encoded by adenovirus vectors as a result of skin-targeted non-invasive gene delivery.

To test whether this technique might be generally applicable, AdCMV-hgmcsf (an adenovirus vector encoding the human granulocyte macrophage colony stimulating factor (hGM-CSF)) was applied onto the skin. To detect antibodies against the human GM-CSF protein, the animal was vaccinated by skin-targeted non-invasive delivery of 10⁸ pfu of AdCMV-hgmcsf. Purified human GM-CSF protein (CalBiochem) separated in a 15% SDS-polyacrylamide gel was transferred to membranes and allowed to react with diluted serum. Other treatments were carried out as described in Figure 3a. Referring to Figure 3b, lane 1, 0.25 µg of human GM-CSF; lane 2, 0.25 µg of BSA; lane 3, 10⁷ pfu of adenovirus. The replication-defective human adenovirus serotype 5 derived AdCMV-hcea and AdCMV-hgmcsf were produced in human 293 cells. A cassette containing the human CEA gene or the human GM-CSF gene, driven by the cytomegalovirus (CMV) early enhancer-promoter element was inserted in place of the E1a deletion. Since the sequences in the E1a region were deleted, the ability of these viruses to replicate autonomously in nonpermissive cells was impaired.

Results (Tang et al., 1997) show that 96% (23/24) of the C57BL/6 strain mice produced antibodies against the human CEA protein a month after skin-targeted non-invasive delivery of AdCMV-hcea, and 43% (6/14) of the same strain mice produced antibodies against the human GM-CSF protein after skin-targeted non-invasive delivery of AdCMV-hgmcsf. Both pre-immune sera collected before NIVS and sera from naive animals failed to react with the human CEA and GM-CSF proteins. The possibility of oral vaccination by ingesting vectors through grooming was eliminated by (1) rinsing vectors away from the skin before animals recovered from anesthesia, (2) pipetting vectors onto unshaved skin, and (3) mixing naive and vaccinated animals in the same cage. No cross-vaccination between naive and vaccinated mice was ever observed, and shaving appeared as an essential component for NIVS, presumably due to the mechanical removal of cornified epithelium along the shaving path. Thus, adenovirus-mediated NIVS is capable of eliciting a humoral immune response against an antigen encoded the vector.

### Reference EXAMPLE 4

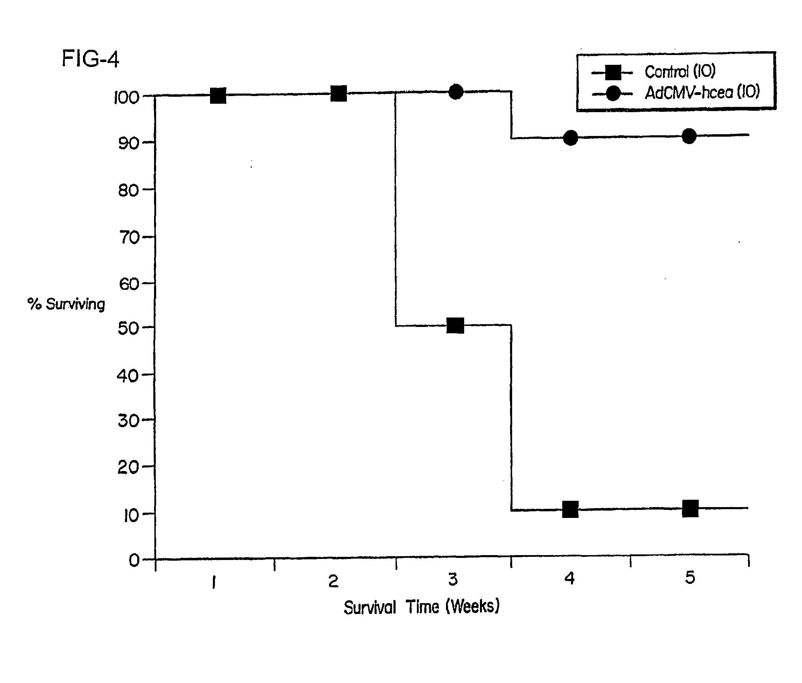

To demonstrate that the techniques of the present invention can elicit a protective antitumor immune response, syngeneic tumor cells that express the human carcinoembryonic antigen (CEA) gene (MC38-CEA-2) (Conry et al., 1995) were inoculated into naive C57BL/6 strain mice and the same strain mice that had been vaccinated by topical application of an adenovirus vector encoding the human CEA gene (AdCMV-hcea). Animals subjected to tumor challenges were observed for survival (Figure 4). In the control group, 90% (9/10) of the animals developed palpable tumor nodules and died within 30 days after tumor cell implantation. In the vaccinated group, only 10% (1/10) of the animals died, and 70% (7/10) of them remained totally tumor-free. Mice were euthanized when the tumor exceeded 1 cm in diameter. The interval between tumor cell injection and euthanization is used as the individual survival time. Referring to Figure 4, control mice (no vaccines were administered) and animals immunized by NIVS (10⁸ pfu of AdCMV-hcea were topically applied a month before) were subjected to tumor challenges. Numbers in parentheses represent the number of animals for each treatment. Results show that non-invasive delivery of genetic vaccines onto the skin may be able to elicit protective immune responses against tumor cells expressing a specific antigen.

### Reference EXAMPLE 5

### Construction of recombinant adenovirus vectors encoding cytokine and co-stimulatory genes

Adenovirus vectors encoding co-stimulatory and cytokine genes were constructed for the co-delivery of these immune-modulatory genes with antigen genes into skin cells in an attempt to direct the immune profile in vaccinated animals. The adenovirus vector AdCMV-mB7.1 encoding the murine B7-1 gene and the adenovirus vector AdCMV-mgmcsf encoding the murine GM-CSF gene were constructed by homologous recombination between two transfected plasmids in human 293 cells following a standard procedure for generating new adenovirus vectors (Gomez-Foix et al., 1992). All transgenes in these vectors were transcriptionally driven by the CMV early enhancer-promoter element. AdCMV-mB7.1 was characterized by staining transduced human lung carcinoma SCC-5 cells with the anti-CD80 antibody (PharMingen), followed by flow cytometric analysis. AdCMV-mgmcsf was characterized by measuring murine GM-CSF secreted from transduced SCC-5 cells with an ELISA kit (Amersham).

### Reference EXAMPLE 6

### Detection of antitumor immunity by in vivo cytotoxicity assay

An *in vivo* cytotoxicity assay was developed in which target cells were implanted as monolayers onto the muscle tissue of mice (Tang et al., 1996). Implantation of target cells as monolayers allowed for an efficient retrieval of target cells for assessing their fates after a few days of *in vivo* growth. This assay was particularly useful for detecting weak immune responses that are not potent enough for eradicating target cells. Immune responses can be characterized by histological analysis of the implantation bed. Without an immune response, target cells would grow. With a potent immune response, target cells would be eradicated in the presence of a large number of immune effector cells at the implantation bed, probably by virtue of migration to and *in situ* sensitization around growing target cells. With a weak immune response, growing target cells would intermingle with infiltrating immune effector cells at the implantation bed. Implanting 5X10⁵ RM1-luc cells (RM1 prostate tumor cells expressing the luciferase gene) as a monolayer into naive C57BL/6 mice resulted in a tumor layer due to proliferation of RM1-luc cells *in vivo,* with no evidence of immune intervention. In contrast to control animals, RM1-luc cells were intermingled with a large number of immune effector cells at the implantation bed in animals vaccinated by skin-targeted non-invasive delivery of AdCMV-luc.

### Reference EXAMPLE 7

### Characterization of immune effector cells recruited by tumor cells

The *in vivo* cytotoxicity assay was able to concentrate a large number of immune effector cells at the implantation bed by implanting a small number of target cells as a monolayer onto muscle. Characterization of specific immune effector cells at the implantation bed may provide evidence as to whether a cell-mediated immune response has been elicited for killing target cells. For characterizing T cells that were recruited by luciferase-expressing tumor cells in animals vaccinated by skin-targeted non-invasive delivery of AdCMV-luc, tissue sections of the implantation bed were stained with an anti-CD3 monoclonal antibody (mAb). RM1-luc cells were produced by lipofecting pHBA-luc DNA into RM1 prostate tumor cells (provided by T. Thompson at the Baylor College of Medicine), followed by selection in medium containing G418. Clones expressing luciferase were characterized by luciferase assay. Five X 10⁵ RM1-luc cells were implanted as a monolayer into a mouse that had been vaccinated by skin-targeted non-invasive delivery of 10⁸ pfu AdCMV-luc. Five days after implantation, the implantation bed was frozen in O.C.T. and sections were cut at 4 µm, dried in 100% acetone, and stained with an anti-CD3 mAb (clone F500A2, provided by P. Bucy at UAB), via the ABC immunoperoxidase procedure with diaminobenzidine as the chromogen.

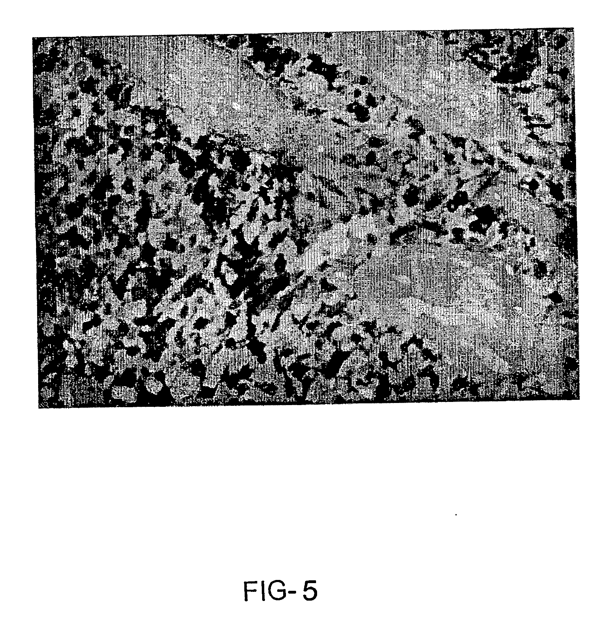

As shown in Figure 5, a large number of T cells infiltrated into the implantation bed after 5 days of *in vivo* growth of RM1-luc cells in a mouse vaccinated by skin-targeted non-invasive delivery of AdCMV-luc (x150) while only a few T cells were found in naive animals. It appeared that the same number of RM1-luc target cells could recruit more T lymphocytes to the implantation bed in vaccinated animals than in naive animals.

For characterizing CTLs that were recruited by target cells, frozen sections of the implantation bed were subjected to *in situ* hybridization using an antisense granzyme A RNA molecule as the probe. Five X 10⁵ RM1-luc cells were implanted as a monolayer into either a naive C57BL/6 mouse or a mouse that had been vaccinated by skin-targeted non-invasive delivery of 10⁸ pfu AdCMV-luc. Five days after implantation, the implantation bed was frozen in O.C.T. and sections were cut at 4 µm. Frozen sections were fixed in 3% paraformaldehyde, incubated in 0.2 M HCl for inhibiting endogenous alkaline phosphatase activity, and hybridized with a heat-denatured antisense granzyme A RNA probe. Probes for *in situ* hybridization were single-stranded RNA molecules produced by transcription from a plasmid containing bacteriophage promoters. During the transcription, digoxigenin-UTP was directly incorporated into the sequence. Sense sequence probes were used as negative controls. After hybridizing with probes, sections were washed and incubated with alkaline phosphatase-conjugated anti-digoxigenin antibody, followed by incubation in the NBT/BCIP enzyme substrate solution.

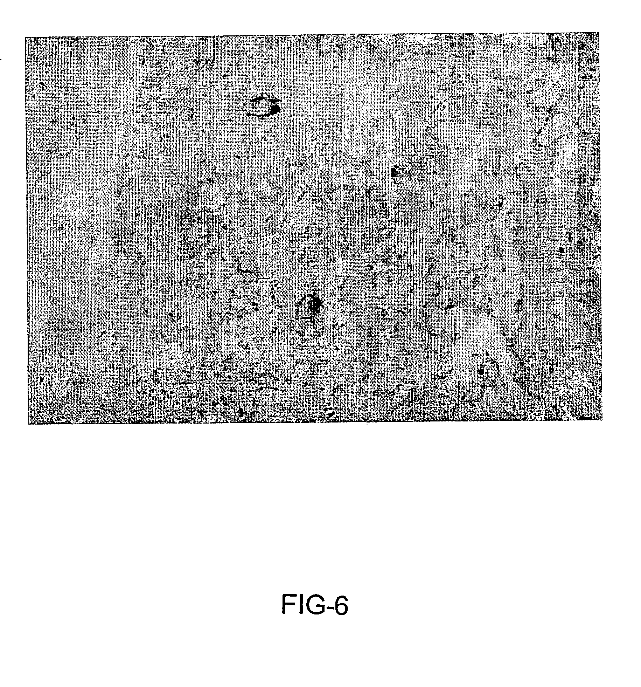

CTLs that express granzyme A are activated CTLs and have been used as predictive markers for tissue rejection during transplantation. Granzyme-positive CTLs were found within the RM1-luc implantation bed only in animals that had been vaccinated by skin-targeted non-invasive delivery of AdCMV-luc (Figure 6). Their presence at the bed suggests that a cell-mediated immune response against tumor cells expressing a specific antigen may have been induced by NIVS.

### REFERENCE EXAMPLE 8

### Topical applications of genetic vaccines by adhesive bandages

It was demonstrated, for the first time, that bandages could be used for the administration of vaccines. This development may allow personnel without medical training to deliver a uniform dose of non-invasive vaccines onto the skin. To transduce skin by bandage, 50 µl of the AdCMV-luc vector described in Example 7 was pipetted into the pad of an adhesive bandage (Johnson & Johnson). The vector-containing bandage was subsequently adhered to pre-shaved skin of a mouse. The vector was kept in contact with naked skin for 18 hours. To detect transgene expression from genetic vectors delivered by a bandage, the skin was assayed for luciferase (Table 1). While the results show substantial variation, transgene expression in the skin was achievable using adhesive bandages.

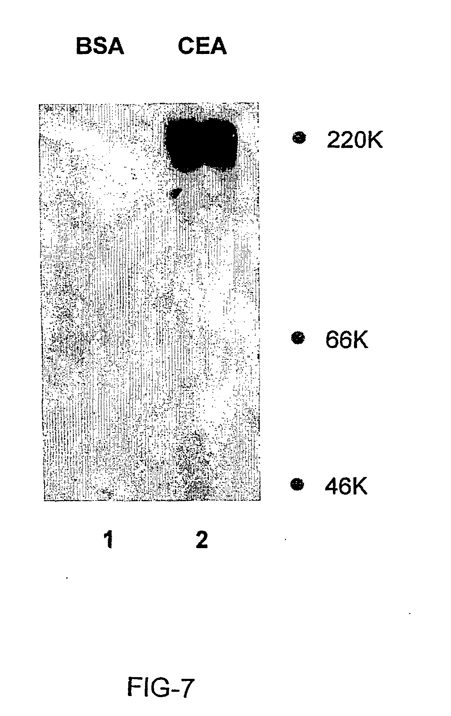

To demonstrate that animals could be vaccinated with non-invasive adhesive bandages, sera from tail bleeds were assayed for anti-CEA antibodies two months after adhering bandages containing AdCMV-hcea onto the skin of mice. As shown in Figure 7 anti-CEA antibodies were detected in 100% (10/10) of mice that received non-invasive vaccines through adhesive bandages.

### REFERENCE EXAMPLE 9

### DNA/adenovirus-mediated NIVS

Adenovirus-based vectors can be made more versatile by binding plasmid DNA to the exterior of an adenovirus. The resulting vector system mediates high-efficiency gene delivery to a wide variety of target cells. This approach allows greatly enhanced flexibility in terms of the size and design of foreign genes. DNA/adenovirus complexes may thus be able to deliver antigen genes into the skin via the same adenovirus receptor-mediated endocytosis pathway with more flexibility -

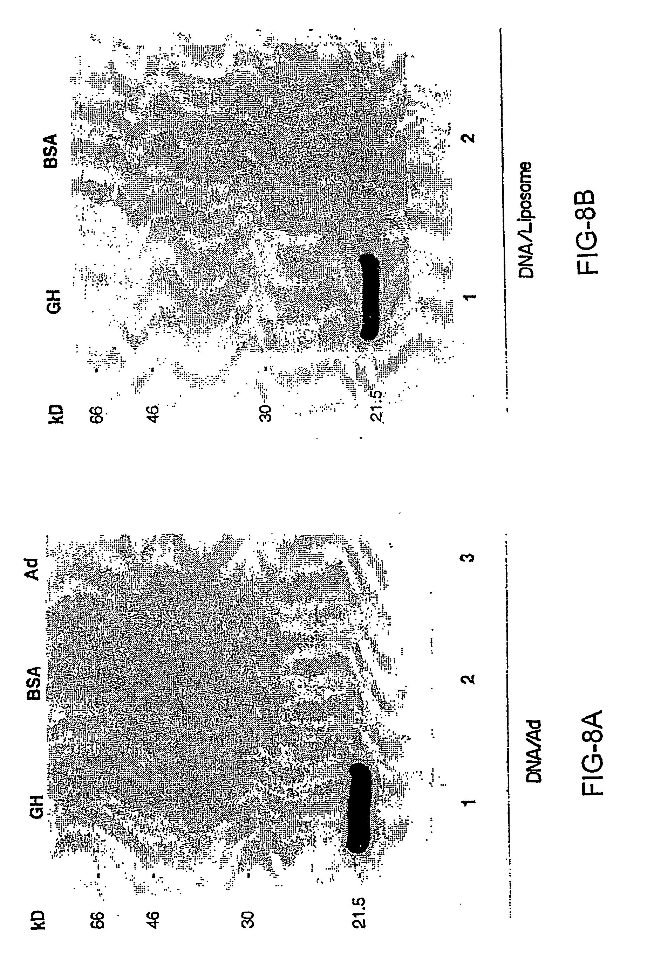

To demonstrate the feasibility of DNA/adenovirus-mediated NIVS, plasmid DNA encoding the human growth hormone (pCMV-GH) (Tang et al., 1992) was allowed to complex with an E4-defective adenovirus. Mice (strain C57BL/6) were vaccinated by contacting DNA/adenovirus complexes with naked skin for one day. Immunized animals were subsequently monitored for the production of antibodies against the human growth hormone protein (hGH) by assaying sera from tail-bleeds. As shown in Figure 8a, lane 1, hGH (0.5 µg); lane 2, BSA (0.5 µg), the test sera reacted in western blots with purified hGH, but not with irrelevant proteins. Of ten mice vaccinated by DNA/adenovirus complexes, eight (80%) produced antibodies against hGH within three months, indicating that specific antibodies could be produced against exogenous proteins encoded by plasmid DNA that is complexed with adenovirus and administered in a non-invasive mode. Pre-immune sera collected before treatment, sera from untreated animals, and sera from animals vaccinated with irrelevant vectors all failed to react with hGH. Thus, DNA/adenovirus complexes, like adenovirus recombinants, appear as a legitimate vector system for NIVS.

### REFERENCE EXAMPLE 10

### DNA/liposome-mediated NIVS

In addition to developing genetic vectors involving adenovirus as carriers for non-invasive vaccines, it has also been demonstrated that mice could be vaccinated by topical application of DNA/liposome complexes without viral elements. It is apparent that many different vectors can be applied in a creative way for the administration of skin-targeted non-invasive vaccines. As shown in Figure 8b, lane 1, hGH (0.5 µg); lane 2, BSA (0.5 µg), the test serum from a mouse immunized by topical application of DNA/liposome complexes encoding hGH reacted with hGH but not with BSA. Of 10 mice vaccinated by DNA/liposome complexes, the test sera reacted with purified hGH in 9 (90%) treated animals within 5 months. Thus, the DNA/liposome complex, like the adenovirus and the DNA/adenovirus complex, appears as another legitimate vector system for NIVS.

### Reference EXAMPLE 11

### Co-expression of DNA-encoded and adenovirus-encoded transgenes

Strategies of augmenting the immune system's response can potentially improve the clinical outcomes of vaccines. Local production of immune-modulatory molecules involved in the activation and expansion of lymphocyte populations may significantly improve the vaccination effects. Adenovirus vectors encoding the murine B7-1 and GM-CSF genes have been made. Topical application of DNA/adenovirus complexes may thus be able to co-express DNA-encoded antigens or immune modulatory molecules with adenovirus-encoded antigens or immune modulatory molecules in individual skin cells for enhancing the immune response against the antigen.

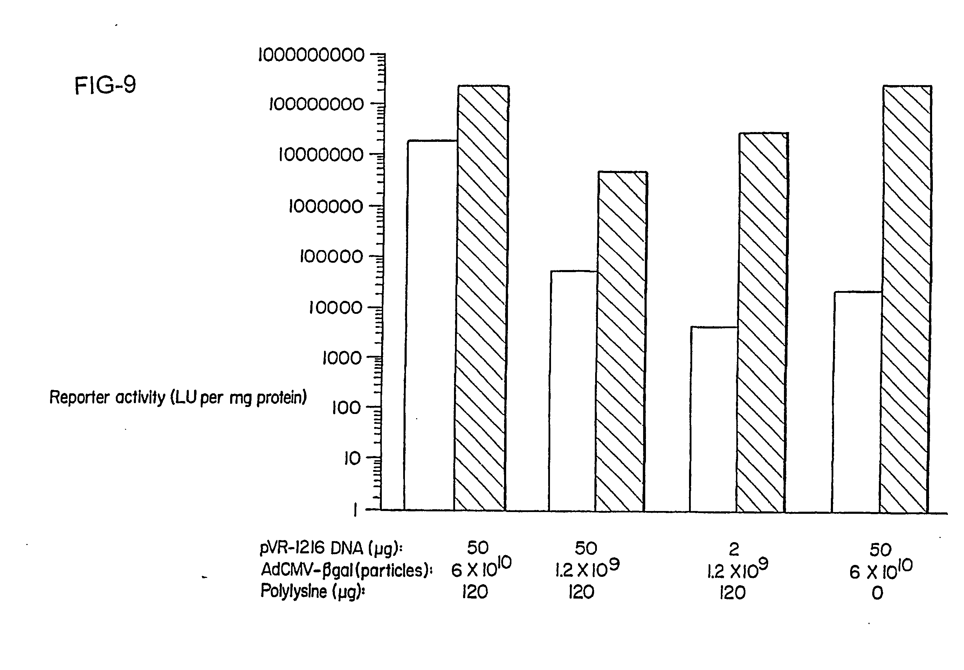

Figure 9 shows that the expression of trans genes from plasmid DNA in target cells is dependent upon the presence of adenovirus, thus allowing plasmid-encoded and adenovirus-encoded transgenes to be co-expressed in the same cell pVR-1216 plasmid DNA (provided by Vical), AdCMV-βgal particles and polylysine were mixed at specific ratios as shown in the figure. The complex was applied to 2 X 10⁵ SCC-5 cells in a well and incubated for 2 hours. The complex was then removed and cells were harvested for luciferase and β-galactosidase assays the next day. Open column: luciferase activity; solid column: β-galactosidase activity. Results show that DNA-encoded transgenes are not expressed in target cells in the absence of adenovirus, whereas adenovirus-encoded transgenes can be expressed in the presence of DNA. It is also possible that DNA may be condensed onto the surface of other viruses for targeting different cell types. Accordingly, this protocol provides a simple but versatile gene delivery system which allows the expression of transgenes from both a virus recombinant and an externally-bound plasmid, simultaneously.

### Reference EXAMPLE 12

### Relative transgene expression in the skin from different genetic vectors by topical application

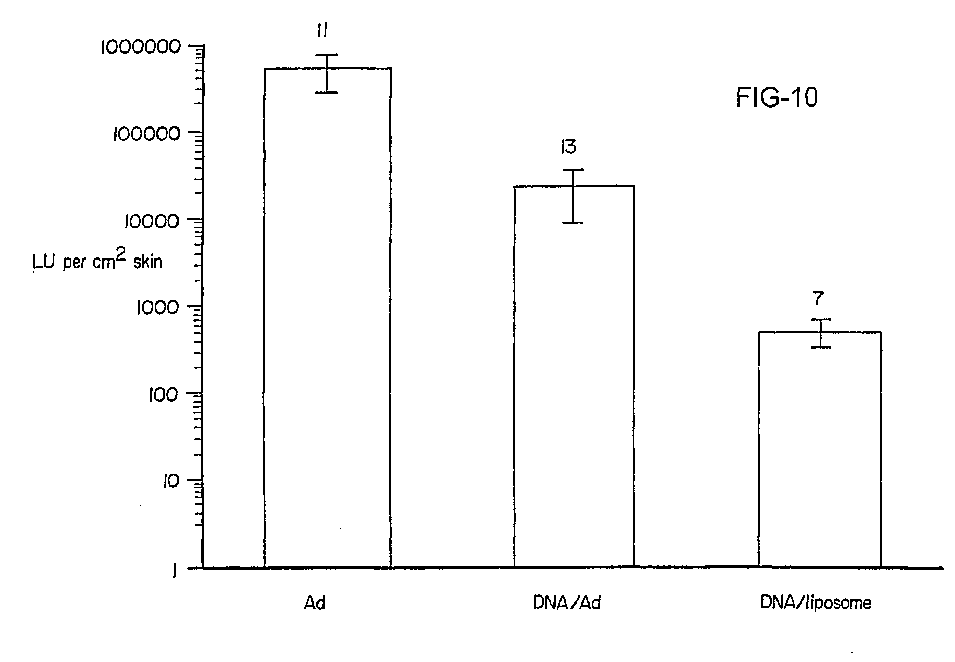

It has been shown that adenovirus recombinants, DNA/adenovirus complexes, DNA/liposome complexes, and perhaps many other genetic vectors can all be applied as carriers for non-invasive vaccines. It is conceivable that the higher the efficiency for transgene expression, the more powerful the carrier will be. To define the relative efficiencies for the vectors utilized, adenovirus recombinants, DNA/adenovirus complexes, or DNA/liposome complexes were allowed to contact mouse skin by topical application for 18 hr. The treated skin was subsequently removed from the animal and assayed for luciferase activity with a luminometer by measurement of integrated light emission for 2 min using the Promega's luciferase assay system, and background was subtracted from the readings. As shown in Figure 10, adenovirus recombinants were found to be the most efficient vector system for skin-targeted non-invasive gene delivery. Mice mock-treated produced no detectable luciferase activity in the skin. LU, light units; Ad, AdCMV-luc; DNA/Ad, pVR-1216 DNA complexed with Ad d11014; DNA/liposome, pVR-1216 DNA complexed with DOTAP/DOPE. Results are the mean log(LU per cm² skin) ± SE (*n* is shown on top of each column). Although the efficiency of DNA/adenovirus complex is lower than that of adenovirus recombinant, it is significantly higher than that of DNA/liposome complex. In addition, adenovirus may be inactivated by UV or gamma irradiation before complexing with DNA to prevent viable viral particles from disseminating. Thus, DNA/adenovirus complexes may appear as the most promising carrier system for the delivery of non-invasive vaccines when efficiency and safety factors are both considered in formulating a new generation of vaccines.

### EXAMPLE 13

### Construction of an expression vector encoding_influenza antigens

An E1/E3-defective adenovirus recombinant encoding the A/PR/8/34 HA gene (AdCMV-PRB.ha) was constructed as described (Gomez-Foix et al., 1992). Briefly, an 1.8 kb BamHI fragment containing the entire coding sequence for HA was excised from the plasmid pDP122B [American Type Culture Collection (ATCC)] and subsequently inserted into the BamHl site of pACCMV.PLPA in the correct orientation under transcriptional control of the human cytomegalovirus (CMV) early promoter. The resulting plasmid encoding HA was co-transfected with the plasmid pJM17 into human 293 cells for generating E1/E3-defective adenovirus recombinants. An E1/E3-defective adenovirus recombinant encoding the A/PR/8/34 nuclear protein (NP) gene (AdCMV-PRB.np) was constructed by cloning the NP gene (provided by Merck) into pACCMV.PLPA, followed by homologous recombination in 293 cells as described above.

A plasmid expression vector encoding HA (pCMV-PR8.ha) and another encoding NP (pCMV-PR8.np) were constructed by cloning the HA and NP genes into pVR1012 (provided by Vical), respectively.

### EXAMPLE 14

### Anti-influenza antibodies generated by topical application and intranasal inoculation of adenovirus based vaccines in mice

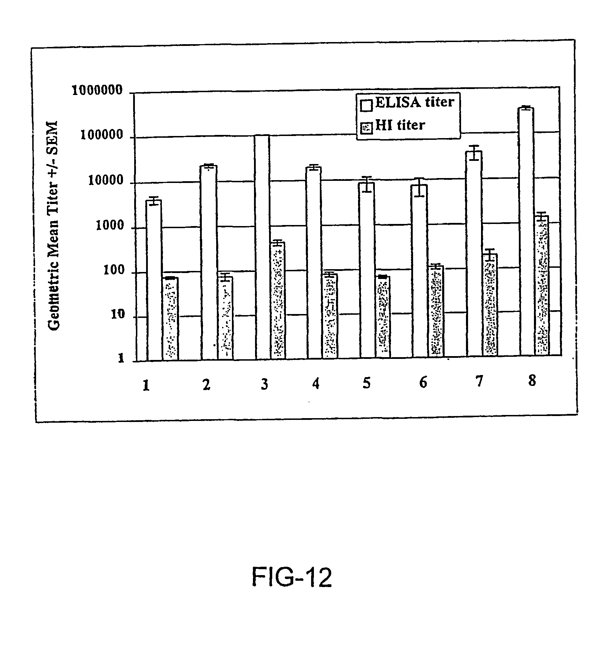

As shown in figure 12, BALB/c mice (3 months old) were immunized by a variety of vaccination modalities including intramuscular injection of DNA, intranasal inoculation of adenovirus vectors, and topical application of an adenovirus-based vaccine patch. Skin-targeted noninvasive vaccination was carried out by pipetting adenovirus vectors onto pre-shaved abdominal skin followed by covering the vector as a thin film over naked skin with a piece of the Tegaderm patch (3M). Unabsorbed vectors were washed away in an hour. All animals were immunized 3 times every 3 weeks. Serum samples were assayed for anti-influenza antibodies 1 week after the last boost. Titers of anti-influenza IgG were determined by ELISA as described (12) using purified A/PR/8/34 virus as the capture antigen. Serum samples and peroxidase-conjugated goat anti-mouse IgG (Promega) were incubated sequentially on the plates for 1 hour at room temperature with extensive washing between each incubation. The end-point was calculated as the dilution of serum producing the same OD₄₉₀ as a 1/100 dilution of preimmune serum. Sera negative at the lowest dilution tested were assigned endpoint titers of 100. The post-immune sera also reacted with a control antigen (e.g., BSA) at a low level. Hemagglutination inhibition (HI) assay was carried out for measuring the ability of anti-HA antibodies to inhibit the agglutination of red blood cells (RBC) by virus, possibly by blocking cell surface binding. Serum samples preabsorbed with chicken RBCs were diluted and mixed with 4 HA units of influenza A/PR/8/34. Chicken RBCs were then added to a final concentration of 0.5%. Agglutination was determined by visual examination. The titer was defined as the dilution being the limit of inhibition. All preimmune sera had titers of ≤20. Group 1, intranasal inoculation of 2.5X10⁷ pfu wild-type adenovirus serotype 5 followed by topical application of 10⁸ pfu AdCMV-PR8.ha and 10⁸ pfu AdCMV-PR8.np 2 weeks later (n=9); Group 2, intranasal inoculation of 2.5X10⁷ pfu wild-type adenovirus serotype 5 followed by intramuscular injection of 100 µg pCMV-PR8.ha DNA and 100 µg pCMV-PR8.np DNA 2 weeks later (n=10); Group 3, intranasal inoculation of 2.5X10⁷ pfu wild-type adenovirus serotype 5 followed by intranasal inoculation of 2.5X10⁷ pfu AdCMV-PR8.ha and 2.5X10⁷ pfu AdCMV-PR8.np 2 weeks later (n=8); Group 4, topical application of 10⁸ pfu AdCMV-PR8.ha and 10⁸ pfu AdCMV-PR8.np (n=10); Group 5, topical application of 10⁸ pfu AdCMV-PR8.np (n=10); Group 6, topical application of 10⁸ pfu AdCMV-PR8.ha (n=10); Group 7, intramuscular injection of 100 µg pCMV-PR8.ha DNA and 100 µg pCMV-PR8.np DNA (n=10); Group 8, intranasal inoculation of 2.5X10⁷ pfu AdCMV-PR8.ha and 2.5X10⁷ pfu AdCMV-PR8.np (n=9). The data was plotted as geometric mean endpoint titers. In the naïve control group (n=7), no anti-influenza antibodies were detectable. The analysis of variance (ANOVA) approach was utilized to compare the differences in ELISA and HI titers. Multiple pairwise comparisons were made with Tukey's procedure with the overall alpha level set at 0.05. The analyses were performed in log scale of the measurements to meet the constant variance assumption required by the ANOVA approach. The differences in ELISA and HI titers among the 8 groups were significant (P<0.0001). The ELISA titer in group 8 was significantly higher than that in other groups (P<0.02). The average ELISA titer in group 1 was the lowest but was not significantly different from that in group 5 or 6. The HI titer in group 8 was the highest and that in group 3 was the second highest. The HI titer values in groups 1, 2, 4, 5, and 6 were not significantly different.

### EXAMPLE 15

### Protection of mice from death following virus challenge

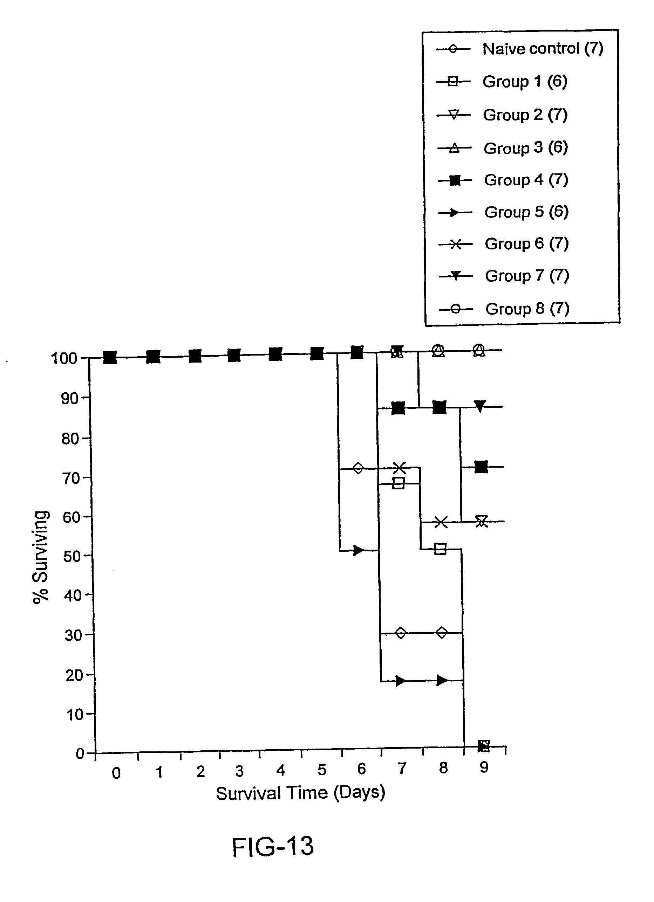

As shown in Figure 13, BALB/c mice (3 months old) were immunized by a variety of vaccination modalities including intramuscular injection of DNA, intranasal inoculation of adenovirus vectors, and topical application of an adenovirus-based vaccine patch. Skin-targeted noninvasive vaccination was carried out by pipetting adenovirus vectors onto pre-shaved abdominal skin followed by covering the vector as a thin film over naked skin with a piece of the Tegaderm patch (3M). Unabsorbed vectors were washed away in an hour. All animals were immunized 3 times every 3 weeks. One week after the last boost, mice were challenged intranasally with a lethal dose of influenza virus A/PR/8/34 (1,000 HA units) and monitored daily for survival. The data was plotted as % survival versus days after challenge. Naive Control, naïve mice without exposure to adenovirus; Group 1, intranasal inoculation of 2.5X10⁷ pfu wild-type adenovirus serotype 5 followed by topical application of 10⁸ pfu AdCMV-PR8.ha and 10⁸ pfu AdCMV-PR8.np 2 weeks later; Group 2, intranasal inoculation of 2.5X10⁷ pfu wild-type adenovirus serotype 5 followed by intramuscular injection of 100 µg pCMV-PR8.ha DNA and 100 µg pCMV-PR8.np DNA 2 weeks later; Group 3, intranasal inoculation of 2.5X10⁷ pfu wild-type adenovirus serotype 5 followed by intranasal inoculation of 2.5X10⁷ pfu AdCMV-PR8.ha and 2.5X10⁷ pfu AdCMV-PR8.np 2 weeks later; Group 4, topical application of 10⁸ pfu AdCMV-PR8.ha and 10⁸ pfu AdCMV-PR8.np; Group 5, topical application of 10⁸ pfu AdCMV-PR8.np; Group 6, topical application of 10⁸ pfu AdCMV-PR8.ha; Group 7, intramuscular injection of 100 µg pCMV-PR8.ha DNA and 100 µg pCMV-PR8.np DNA; Group 8, intranasal inoculation of 2.5X10⁷ pfu AdCMV-PR8.ha and 2.5X10⁷ pfu AdCMV-PR8.np. AdCMV-PR8.ha, an adenovirus vector encoding the A/PR/8/34 hemagglutinin; AdCMV-PR8.np, an adenovirus vector encoding the A/PR/8/34 nuclear protein; pCMV-PR8.ha, a plasmid expression vector encoding the A/PR/8/34 hemagglutinin; pCMV-PR8.np, a plasmid expression vector encoding the A/PR/8/34 nuclear protein. Numbers in parentheses represent the number of animals for each treatment.

Pre-exposure to wild-type adenovirus did not intervene with this mode of vaccination. As shown in the Phase I Final Report, high levels of neutralizing antibody against influenza A/PR/8/34 could be elicited by intranasal inoculation of these vectors even in animals with pre-exposure to adenovirus. Results suggested that protection may be mediated principally by a humoral immune response when animals were immunized by intranasal inoculation of adenovirus recombinants. In contrast to the intranasal route, animals immunized by topical application of AdCMV-PR8.ha and AdCMV-PR8.np were afforded 7 1 % protection from the challenge. However, animals with pre-exposure to adenovirus failed to be protected by NIVS (noninvasive vaccination onto the skin). As shown in the Phase I Final Report, animals immunized by NIVS produced a relatively low level of neutralizing antibody against influenza A/PR/8/34. Like antibodies induced by intranasal vaccines, production of anti-influenza antibodies as provoked by NIVS was not intervened by pre-exposure to adenovirus. Results suggested that protective immunity may be mediated by a cellular immune response when animals were immunized by NIVS, and this immunologic mechanism could be suppressed by pre-existing immunity to the vector. For patients who can take intranasal vaccines without complications due to any respiratory problems, it may thus be appropriate to co-administer adenovirus-based epicutaneous vaccines together with their intranasal counterparts in an attempt to activate both arms of the immune system simultaneously. There is also an urgent need to develop a non-immunogenic vaccine carrier for NIVS in order to vaccinate animals with pre-existing immunity to adenovirus.

### Reference EXAMPLE 16

### Elicitation of anti-HA antibodies in a pigtail macaque by NIVS

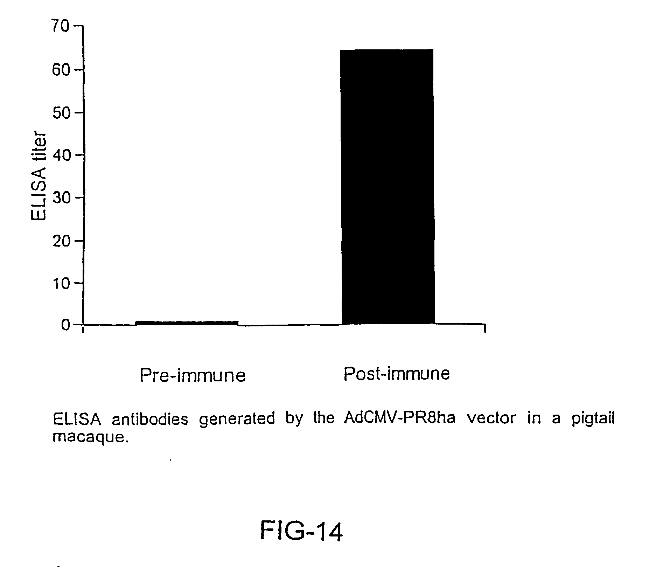

Although NIVS could reproducibly elicit systemic immune responses in mice (Figures 12 and 13), it may not be possible for NIVS to immunize humans if transdermal diffusion of vectors should be required for vaccination to occur, because human skin is thicker than its murine counterpart. However, non-invasive vaccine patches may be able to immunize humans or other animals with thick skin if all that is required is a transient but productive wave of antigen expression in cells within the outer layer of skin. To address these issues, we have immunized a pigtail macaque by AdCMV-PR8.ha in a non-invasive mode. As shown in Figure 14, the immunized animal produced antibodies against HA in 4 weeks. The result provides evidence that non-invasive vaccine patches may be able to immunize many different species in addition to mice.

In Figure 14, a pigtail macaque was immunized in a non-invasive mode by pipetting 10¹⁰ pfu of AdCMV-PR8.ha onto pre-shaved abdominal skin followed by covering the vector as a thin film over naked skin with the Tegaderm patch (3M). Unabsorbed vectors were washed away in 5 hours. Serum samples were assayed for anti-HA antibodies at 4 weeks after inoculation. Titers of anti-HA IgG were determined by ELISA using purified A/PR/8/34 virus as the capture antigen. Serum samples and peroxidase-conjugated goat anti-monkey IgG (Bethyl Laboratories, Inc.) were incubated sequentially on the plates for 1 hour at room temperature with extensive washing between each incubation. The end-point was calculated as the dilution of serum producing the same OD₄₉₀ as a 1/100 dilution of preimmune serum. Sera negative at the lowest dilution tested were assigned endpoint titers of 1.

### Reference EXAMPLE 17

### Relocation of luciferase spots in the skin after localized gene delivery in a non-invasive mode

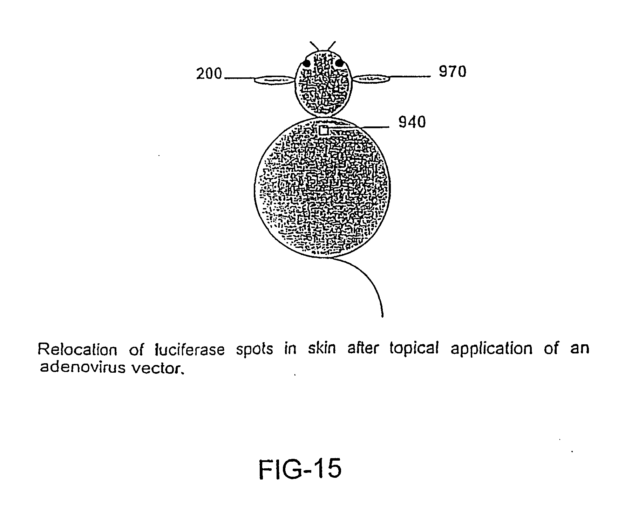

In an attempt to determine whether antigen genes delivered onto the surface of the skin could diffuse into deep tissues and express antigens in cells beyond epidermis, we incubated neck skin with AdCMV-luc (an adenovirus vector encoding luciferase) (Tang et al., 1997). As shown in Figure 15, luciferase activity could be detected in ears (or as discrete luciferase spots in other areas within the skin) in some of the treated animals one day after non-invasive delivery of AdCMV-luc onto neck skin. Luciferase was undetectable in any of the internal organs including lymph nodes, liver, spleen, heart, lung and kidney.

In Figure 15, 1 x 10⁸ pfu of AdCMV-luc was incubated with neck skin for an hour. Neck skin as well as ears were harvested for luciferase assay as described (Tang et al., 1994) one day after inoculation. Numbers represented light units with background subtracted from the readings.

In a further attempt to identify and characterize the target cells that are able to express the transgene from a topically-applied adenovirus vector, and the putative mobile cells containing the protein expressed from the transgene, we stained skin sections with X-gal after topical application of AdCMV-βgal (an adenovirus vector encoding β-galactosidase) (Tang et al., 1994). By examining histological sections in search of dark blue cells, we identified labeled hair matrix cells within hair follicles and labeled keratinocytes in the outermost layer of epidermis as the principal target cells for adenovirus-mediated transduction when the vector was inoculated in a noninvasive mode (photographs available upon request). None of the dermal fibroblasts were transduced by this procedure, although these cells were highly transducible when AdCMV-βgal was injected intradermally using a needle (photographs available upon request). Results suggested that few, if any, of the adenovirus particles that were topically applied could penetrate into dermis beyond the outer layer of epidermis. Microscopic examination of histologic sections did not reveal any physical abrasions of the transduced skin. Macroscopically, there was no inflammation associated with the treated skin. However, transduced cells could only be visualized within the inoculation area (*e.g*., neck skin). We were unable to identify dark blue cells in ears or other areas within the skin when luciferase activities could be detected in those areas (Figure 4), probably because luciferase assay is more sensitive than X-gal-mediated β-galactosidase assay. We hypothesize that some antigen-presenting cells (APCs) may respond to antigens expressed on the surface of the skin by acquiring the antigen. The protein may be degraded rapidly, hence undetectable from internal organs including lymph nodes. The biological significance of this relocation of antigens within the skin is unknown.

### Reference EXAMPLE 18

### Amplification of foreign DNA in various tissues after localized gene delivery in a noninvasive mode.

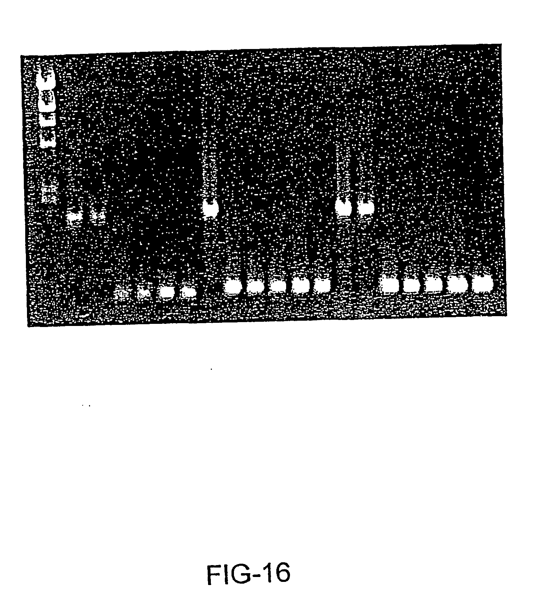

In an attempt to determine whether topical application of an adenovirus vector can also deliver exogenous DNA beyond the inoculation area, we extracted DNA from various tissues, followed by amplification of the transgene as well as the adenovirus type 5 fiber gene by PCR after noninvasive delivery of AdCMV-PR8.ha onto skin. As shown in Figure 16, the full-length HA and fiber genes could be amplified from skin 3 hours post-inoculation. The full-length gene was usually undetectable in skin DNA after 1 day or in DNA extracted from other tissues. However, subfragments of both HA and fiber genes could be amplified from liver, whole blood, ear, abdominal skin, or pooled lymph nodes using different sets of primers. No foreign DNA was detectable in any of the tissues 4 weeks post-inoculation. Results suggested that topical application of an adenovirus vector could deliver exogenous DNA into a localized area in skin, although foreign DNA may be rapidly acquired by some putative antigen-presenting cells, degraded, and relocated into deep tissues. The elimination of foreign DNA in 4 weeks highlights the safety of NIVS. In Figure 16, AdCMV-PR8.ha and AdCMV-luc were inoculated onto preshaved skin in a noninvasive mode. DNA was extracted by DNAZOL (GIBCOBRL), and amplified by the following sets of primers: --
Ha5.1: 5'-ATGAAGGCAAACCTACTGGT-3' (SEQ ID NO:1)
Ha3.1: 5'-GATGCATATTCTGCACTGCA-3' (SEQ ID NO:2)

Ha5.2: 5'-GTGGGGTATTCATCACCCGT-3' (SEQ ID NO:3)
Ha3.2: 5'-TGCATAGCCTGATCCCTGTT-3' (SEQ ID NO:4)

Luc5.1: 5'-GCGCCATTCTATCCTCTAGA-3' (SEQ ID NO:5)
Lug3.1: 5'-ACAATTTGGACTTTCCGCCC-3' (SEQ ID NO:6)

Luc5.2: 5'-GTACCAGAGTCCTTTGATCG-3' (SEQ ID NO:7)
Luc3.2: 5'-CCCTCGGGTGTAATCAGAAT-3' (SEQ ID NO:8)

Fb5.1: 5'-CCGTCTGAAGATACCTTCAA-3' (SEQ ID NO:9)
Fb3.1: 5'-ACCAGTCCCATGAAAATGAC-3' (SEQ ID NO:10)

Fb5.2: 5'-GGCTCCTTTGCATGTAACAG-3' (SEQ ID NO:11)
Fb3.2: 5'-CCTACTGTAATGGCACCTGT-3' (SEQ ID NO:12)

Ha5.1 and Ha3.1 amplified the nearly full-length 1.7 kb HA gene; Ha5.2 and Ha3.2 amplified an 0.6 kb subfragment encompassing 33% of the HA gene; Luc5.1 and Luc3.1 amplified the nearly full-length 1.7 kb luciferase gene; Luc5.2 and Luc3.2 amplified an 0.52 kb subfragment encompassing 30% of the luciferase gene; Fb5.1 and Fb3.1 amplified the nearly full-length 1.7 kb adenovirus type 5 fiber gene; Fb5.2 and Fb3.2 amplified an 0.55 kb subfragment encompassing 32% of the fiber gene. Lane M, Molecular weight marker (Lambda DNA cleaved with HindIII); lane 1, the nearly full-length luciferase gene amplified by Luc5.1 and Luc3.1 from skin DNA 3 hours after NIVS; lane 2, the nearly full-length luciferase gene amplified by Luc5.1 and Luc3.1 from skin DNA 1 day after NIVS; lane 3, a subfragment of luciferase DNA amplified by Luc5.2 and Luc3.2 from mouse ear DNA 1 day after NIVS; lane 4, a subfragment of luciferase DNA amplified by Luc5.2 and Luc3.2 from lymph node DNA 1 day after NIVS; lane 5, a subfragment of luciferase DNA amplified by Luc5.2 and Luc3.2 from liver DNA 1 day after NIVS; lane 6, a subfragment of luciferase DNA amplified by Luc5.2 and Luc3.2 from DNA extracted from whole blood 1 day after NIVS,; lane 7, the nearly full-length HA gene amplified by Ha5.1 and Ha3.1 from skin DNA 3 hours after NIVS; lane 8, a subfragment of HA gene amplified by Ha5.2 and Ha3.2 from skin DNA 1 day after NIVS; lane 9, a subfragment of HA gene amplified by Ha5.2 and Ha3.2 from lymph node DNA 1 day after NIVS; lane 10, a subfragment of HA gene amplified by Ha5.2 and Ha3.2 from liver DNA 1 day after NIVS; lane 11, a subfragment of HA gene amplified by Ha5.2 and Ha3.2 from kidney DNA 1 day after NIVS; lane 12, a subfragment of HA gene amplified by Ha5.2 and Ha3.2 from DNA extracted from whole blood 1 day after NIVS; lane 13, the nearly full-length fiber gene amplified by Fb5.1 and Fb3.1 from skin DNA 3 hours after NIVS; lane 14, the nearly full-length fiber gene amplified by Fb5.1 and Fb3.1 from skin DNA 1 day after NIVS; lane 15, a subfragment of fiber gene amplified by Fb5.2 and Fb3.2 from skin DNA 1 day after NIVS; lane 16, a sub fragment of fiber gene amplified by Fb5.2 and Fb3.2 from ear DNA 1 day after NIVS; lane 17, a subfragment of fiber gene amplified by Fb5.2 and Fb3.2 from lymph node DNA 1 day after NIVS; lane 18, a subfragment of fiber gene amplified by Fb5.2 and Fb3.2 from liver DNA 1 day after NIVS; lane 19, a subfragment of fiber gene amplified by Fb5.2 and Fb3.2 from DNA extracted from whole blood 1 day after NIVS. DNA from lymph nodes was extracted by pooling inguinal, cervical, and brachial lymph nodes in DNAZOL solution. DNA was amplified for 35 cycles at optimized annealing temperatures in a Stratagene Robocycler gradient 40 thermal cycler. Amplified DNA fragments were actionated in 1% agarose gel and stained with ethidium bromide.

### Reference EXAMPLE 19

### A depilatory agent is not required for NIVS

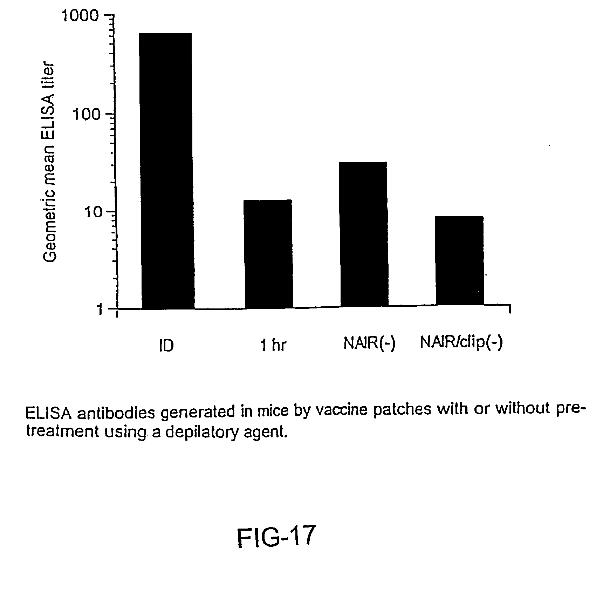

To determine whether a depilatory agent such as NAIR (Tang et al., 1997) is essential for NIVS, we have compared antibody titers elicited by vaccine patches with or without pre-treatment using NAIR. Figure 17 shows that antibody titers in mice without NAIR pre-treatment are as high as their counterparts with NAIR pre-treatment. The elimination of NAIR simplifies the NIVS procedure.

In Figure 17, mice were either injected intradermally (ID) with a dose of 10⁸ pfu, or immunized in a non-invasive mode (NIVS) by pipetting 10⁸ pfu of AdCMV-hcea (Tang et al., 1997) onto abdominal skin followed by covering the vector as a thin film over naked skin with a piece of the Tegaderm patch (3M). Unabsorbed vectors were washed away. Serum samples were assayed for anti-CEA antibodies at 4 weeks after inoculation. Titers of anti-CEA IgG were determined by ELISA using purified human CEA (CalBiochem) as the capture antigen. Serum samples and peroxidase-conjugated goat anti-mouse IgG (Promega) were incubated sequentially on the plates for 1 hour at room temperature with extensive washing between each incubation. The end-point was calculated as the dilution of serum producing the same OD₄₉₀ as a 1/100 dilution of preimmune serum. Sera negative at the lowest dilution tested were assigned endpoint titers of 1. The data was plotted as geometric mean endpoint ELISA titers, where n=4 for ID, n=14 for 1 hr, n=10 for NAIR(-), and n=15 for NAIR/clip(-). ID, intradermal injection; 1 hr, vectors were in contact with the outer layer of skin for an hour with shaving and NAIR pre-treatment; NAIR(-), vectors were in contact with the outer layer of skin overnight with shaving but without NAIR pre-treatment; NAIR/clip(-), vectors were in contact with the outer layer of skin overnight with neither shaving nor NAIR pre-treatment.

### Reference EXAMPLE 20

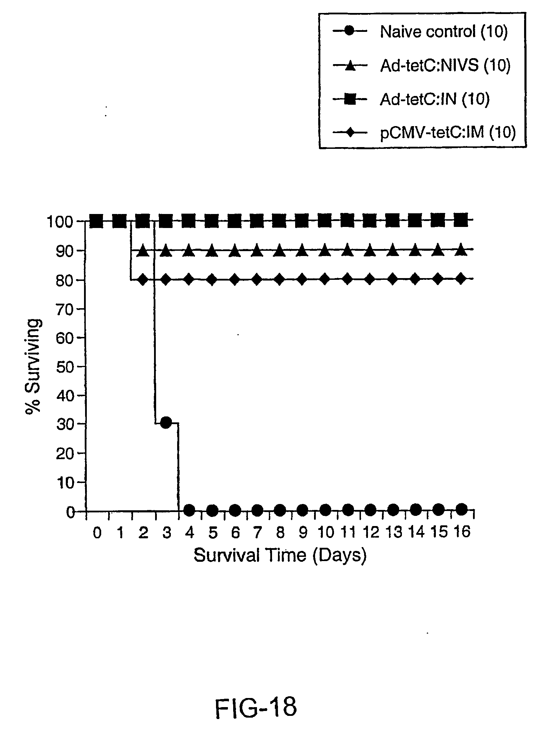

As shown in Figure 18, BALB/c mice (3 months old) were immunized by a variety of vaccination modalities including intramuscular injection of DNA, topical application or intranasal inoculation of an adenovirus-based tetanus vaccine. Skin-targeted noninvasive vaccination was carried out by pipetting approximately 10⁸ pfu AdCMV-tetC onto pre-shaved abdominal skin followed by covering the vector as a thin film over naked skin with a piece of the Tegaderm patch (3M). Unabsorbed vectors were washed away in an hour. Nasal vaccines were administered by pipetting approximately 10⁷ pfu AdCMV-tetC into the nasal cavity. All animals were immunized 3 times every 3 weeks. One week after the last boost, mice were challenged by injecting a lethal dose of *Clostridium tetani* into the footpad and monitored daily for survival. The data was plotted as % survival versus days after challenge. Naïve Control, naïve mice without vaccination prior to challenge. AdtetC:NIVS, mice immunized by topical application of AdCMV-tetC; Ad-tetC:IN, mice immunized by intranasal inoculation of AdCMV-tetC; pCMV-tetC:IM, mice immunized by intramuscular injection of 100 g pCMV-tetC DNA. AdCMV-tetC, an adenovirus vector encoding the *Clostridium tetani* toxin C-fragment; pCMV-tetC, a plasmid expression vector encoding the *Clostridium tetani* toxin C-fragment. Numbers in parentheses represent the number of animals for each treatment.

The herein examples involving nasal administration further illustrate that one can achieve a suitable response via mucosal administration; and, that mucous membranes such as those in the oral cavity can be employed as routes for administration, e.g., buccal and perlingual administration are envisioned by the invention and are demonstrated and discussed via herein examples involving nasal administration, as well as by the general teachings herein.

Thus, the invention includes the application of a recombinant vectored vaccine containing one or more genetic inserts that encode an antigen or epitope of interest or an immune stimulus, or a gene-product from a recombinant vaccine, to the buccal surface of the oral cavity, whereby the product(s) encoded by the inserted gene(s) produce an immunological response that may be protective or therapeutic against an infectious disease. The invention further comprehends such recombinant vectored vaccine or gene-product of a recombinant vaccine incorporated onto, into or adheared to a matrix, forming a carrier mechanism from which the products for immunization may be released onto the buccal surface or into the oral cavity. The invention yet further includes such embodiments wherein the matrix into which the product for immunization is incorporated may be bioactive or inactive and composed of materials which maintain the integrity of the products for immunization; for instance, the matrix material may be composed of polymeric substances such as glucose or other sugars which are biodegradeable, or other biodegradable substances, or materials that are disposable, but may not be biodegradable.

**TABLE 1. Detection of transgene expression from genetic vectors delivered by a bandage, the skin was assayed for luciferase**

| Incubation time (hours) | LU per cm² skin |
|---|---|
| 1 | 0 |
| 1 | 2,100 |
| 2 | 0 |
| 2 | 0 |
| 2 | 6,200 |
| 2 | 7,300 |
| 2 | 13,000 |
| 2 | 48,000 |
| 2 | 1,800 |
| 2 | 13,000 |
| 18 | 830 |
| 18 | 2,400 |
| 18 | 260 |
| 18 | 630 |
| is | 1,300,000 |
| 18 | 24,000 |
| 18 | 2,700 |
| 18 | 280 |

**TABLE 2. Summary of AdCMV-PR8.ha DNA relocation following topical application**

| Time point | Ear pinna | Abdominal skin^{a} | Lymph nodes^{b} | Spleen | Liver | Kidney | Blood | Muscle^{c} |
|---|---|---|---|---|---|---|---|---|
| I. Nearly full-length HA gene | | | | | | | | |
| 3 hr | 0/2 | 2/2 | 0/2 | 0/2 | 0/2 | 0/2 | 0/2 | 0/2 |
| 1 day | 0/3 | 2/3 | 0/3 | 0/3 | 0/3 | 0/3 | 0/3 | 0/3 |
| 1 month | 0/2 | 0/2 | 0/2 | 0/2 | 0/2 | 0/2 | 0/2 | 0/2 |
| | | | | | | | | |
| II. Subfragment of HA gene | | | | | | | | |
| 3 hr | 0/2 | 2/2 | 0/2 | 0/2 | 0/2 | 0/2 | 0/2 | 0/2 |
| 1 day | 1/3 | 3/3 | 3/3 | 1/3 | 2/3 | 2/3 | 2/3 | 2/3 |
| 1 month | 0/2 | 0/2 | 0/2 | 0/2 | 0/2 | 0/2 | 0/2 | 0/2 |
| | | | | | | | | |
| III. Nearly full-length fiber gene | | | | | | | | |
| 3 hr | 0/2 | 2/2 | 0/2 | 0/2 | 0/2 | 0/2 | 0/2 | 0/2 |
| 1 day | 1/3 | 3/3 | 0/3 | 0/3 | 0/3 | 0/3 | 0/3 | 0/3 |
| 1 month | 0/2 | 0/2 | 0/2 | 0/2 | 0/2 | 0/2 | 0/2 | 0/2 |
| | | | | | | | | |
| IV. Sub fragment of fiber gene | | | | | | | | |
| 3 hr | 0/2 | 2/2 | 0/2 | 0/2 | 0/2 | 0/2 | 0/2 | 0/2 |
| 1 day | 1/3 | 3/3 | 0/3 | 0/3 | 0/3 | 0/3 | 0/3 | 0/3 |
| 1 month | 0/2 | 0/2 | 0/2 | 0/2 | 0/2 | 0/2 | 0/2 | 0/2 |

Mice were immunized by topical application of AdCMV-PR8.ha as described in the foregoing Examples and Figures, e.g., description pertaining to Figure 1. At indicated time points, total DNA was extracted from the tissues and amplified by PCR using specific primer sets as described in the foregoing Examples and Figures, e.g., description pertaining to Figure 3. The data were presented as the number of animals containing detectable signals for a specific tissue per total number of animals analyzed. ^{a}Administration site; ^{b}pooled lymph nodes; ^{c}hind leg quadriceps.

**TABLE 3. Summary of pCMV-PR8.ha DNA relocation following intramuscular injection**

| Time point | Ear pinna | Abdominal skin | Lymph nodes^{a} | Spleen | Liver | Kidney | Blood | Muscle^{b} |
|---|---|---|---|---|---|---|---|---|
| I. Nearly full-length HA gene | | | | | | | | |
| 3 hr | 2/3 | 0/3 | 3/3 | 1/3 | 0/3 | 0/3 | 1/3 | 3/3 |
| 1 day | 0/3 | 0/3 | 0/3 | 0/3 | 0/3 | 1/3 | 0/3 | 0/3 |
| 1 month | 0/2 | 0/2 | 0/2 | 0/2 | 0/2 | 0/2 | 0/2 | 0/2 |
| | | | | | | | | |

| II. Subfragment of HA gene | | | | | | | | |
|---|---|---|---|---|---|---|---|---|
| 3 hr | 3/3 | 1/3 | 3/3 | 2/3 | 3/3 | 2/3 | 3/3 | 3/3 |
| 1 day | 2/3 | 1/3 | 2/3 | 1/3 | 3/3 | 2/3 | 2/3 | 3/3 |
| 1 month | 1/2 | 1/2 | 2/2 | 1/2 | 1/2 | 0/2 | 0/2 | 1/2 |

Mice were immunized by intramuscular injection of pCMV-PR8.ha DNA as described in the foregoing Examples and Figures, e.g., description pertaining to Figure 1. At indicated time points, total DNA was extracted from the tissues and amplified by PCR using specific primer sets as described the foregoing Examples and Figures, e.g., description pertaining to Figure 3. The data were presented as the number of animals containing detectable signals for a specific tissue per total number of animals analyzed. ^{a}Pooled lymph nodes; ^{b}hind leg quadriceps (administration site).

**TABLE 4. Summary of AdCMV-PR8.ba DNA relocation following administration of heat-inactivated adenovirus vectors**

| Time point | Ear pinna | Abdominal Skin^{a} | Lymph Nodes^{b} | Spleen | Liver | Kidney | Blood | Muscle^{c} |
|---|---|---|---|---|---|---|---|---|
| I. Nearly full-length HA gene | | | | | | | | |
| 1 day | 0/3 | 1/3 | 0/3 | 0/3 | 0/3 | 0/3 | 0/3 | 0/3 |
| | (3/7) | (7/7) | (1/7) | (0/7) | (0/7) | (0/7) | (0/7) | (0/7) |
| | | | | | | | | |
| II. Subfragment of HA gene | | | | | | | | |
| 1 day | 0/3 | 3/3 | 0/3 | 0/3 | 0/3 | 0/3 | 0/3 | 0/3 |
| | (4/7) | (7/7) | (2/7) | (1/7) | (1/7) | (0/7) | (0/7) | (0/7) |
| | | | | | | | | |
| III. Nearly full-length fiber gene | | | | | | | | |
| 1 day | 0/3 | 2/3 | 0/3 | 0/3 | 0/3 | 0/3 | 0/3 | 0/3 |
| | (2/7) | (6/7) | (1/7) | (0/7) | (1/7) | (0/7) | (0/7) | (0/7) |
| | | | | | | | | |
| IV. Subfragment of fiber gene | | | | | | | | |
| 1 day | 0/3 | 3/3 | 0/3 | 0/3 | 0/3 | 0/3 | 0/3 | 0/3 |
| | (2/7) | (7/7) | (2/7) | (0/7) | (2/7) | (1/7) | (1/7) | (0/7) |

### Summary of AdCMV-PR8.ha DNA relocation following topical application:

AdCMV-PR8.ha particles were inactivated by heating at 95°C for 10 min. Vectors were administered to mice either by topical application as described in the foregoing Examples and Figures, e.g., description pertaining to Figure 1, or by intradermal injection of an equivalent amount of vectors using a needle. One day following localized gene delivery, total DNA was extracted from various tissues. Nearly full-length HA and fiber genes and their subfragment counterparts were amplified by PCR using specific primer sets as described in Figure 3 legend. The data were presented as the number of animals containing detectable signals for a specific tissue per total number of animals analyzed. Numbers without parentheses represent topical application; numbers in parentheses represent intradermal injection. ^{a}Administration site; ^{b}pooled lymph nodes; ^{c}hind leg quadriceps. Significance: It is possible that vector DNA may relocate to distant tissues following topical application by three different mechanisms: (1) translocation across skin by diffusion followed by relocation via circulation, (2) translocation across skin followed by subsequent pinocytotic uptake into antigen-presenting cells (APCs), (3) transduction of keratinocytes followed by intercellular transfer of exogenous biomolecules into APCs. Although heat-inactivated adenovirus vectors are incapable of transducing cells as shown by their failure to produce cytopathic effects (CPE) in human 293 cells probably due to denaturation of essential ligands (*e.g*., CAR and RGD motif), they should still be able to diffuse into the skin as live vectors do if diffusion should occur. Results show that the principal mechanism mediating vector DNA relocation following NIVS was unlikely due to translocation across skin by diffusion. Topical application of adenovirus vectors may thus represent a noninvasive rather than a transdermal vaccination modality.

### REFERENCES

Barry, M.A. et al. Protection against mycoplasma infection using expression-library immunization. Nature 377, 632-635 (1995).
Conry, R.M. et al. A carcinoembryonic antigen polynucleotide vaccine for human clinical use. Cancer Gene Ther. 2, 33-38 (1995).
Cotten, M. et al. High-efficiency receptor-mediated delivery of small and large (48 kilobase) gene constructs using the endosome-disruption activity of defective or chemically inactivated adenovirus particles. Proc. Natl. Acad. Sci USA 89, 6094-6098 (1992).
Glenn, G.M. et al. Skin immunization made possible by cholera toxin. Nature 391, 851 (1998).
Gomez-Foix et al. Adenovirus-mediated transfer of the muscle glycogen phosphorylase gene into hepatocytes confers altered regulation of glycogen metabolism. J.Biol. Chem., 267, 25129-25134 (1992).
Johnston, S. A. & Tang, D.-c. Gene gun transfection of animal cells and genetic immunization. Meth. Cell Biol. 43, 353-365 (1994).
McDonnell, W.M. & Askari, F.K. DNA vaccines. New Engl. J. Med. 334, 42-45 (1996).
Tang, D.-c. et al. Genetic immunization is a simple method for eliciting an immune response. Nature 356, 152-154 (1992).
Tang, D.-c. et al. Butyrate-inducible and tumor-restricted gene expression by adenovirus vectors. Cancer Gene Ther. 1, 15-20 (1994).
Tang, D.-c. et al. In vivo cytotoxicity assay for assessing immunity. J. Immunol. Methods 189, 173-182 (1996).
Tang, D.-c. et al. Vaccination onto bare skin. Nature 388, 729-730 (1997).

## Claims

1. Use of an adenoviral vector for the preparation of a non-invasive vaccine for mucosal administration, wherein the vector contains and expresses an exogenous nucleic acid encoding an influenza hemagglutinin antigen or an epitope thereof, and/or an influenza nuclear protein antigen or an epitope thereof, and the vector is deleted in its E1 and E3 regions, and wherein the non-invasive vaccine for mucosal administration is to be delivered to the nasal mucosa.

2. Use according to claim 1, wherein the expression in an animal of an influenza hemagglutinin antigen or an epitope thereof, and/or an influenza nuclear protein antigen or an epitope thereof, stimulates or modulates an immunological response against influenza.

3. Use according to claim 2, wherein the response is induced by the vector expressing the nucleic acid in cells of the animal.

4. Use according claim 2 or 3, wherein the animal is a vertebrate.

5. Use according to claim 4, wherein the vertebrate is a bird or a mammal.

6. Use according to claim 4, wherein the vertebrate is a human or a companion, a domesticated, a food-or-feed producing, a livestock, a game, a racing or a sport animal.

7. Use according to claim 5, wherein the animal is a cow, a horse, a dog, a cat, a goat, a sheep, a pig, or a chicken, a duck or a turkey.

8. Use according to any one of claims 1 to 7, wherein the adenoviral vector comprises a human adenovirus.

9. Use according to any one of claims 1 to 7, wherein the adenoviral vector comprises a canine adenovirus.

10. Use according to any one of claims 1 to 9, wherein the adenoviral vector comprises a viral vector having all viral genes deleted therefrom.

11. An adenoviral vector which contains and expresses an exogenous nucleic acid encoding an influenza hemagglutinin antigen or an epitope thereof, and/or an influenza nuclear protein antigen or an epitope thereof, and which is deleted in its E1 and E3 regions for use as a non-invasive vaccine for mucosal administration, wherein the non-invasive vaccine for mucosal administration is to be delivered to the nasal mucosa.

12. An adenoviral vector for use according to claim 11, wherein the expression in an animal of an influenza hemagglutinin antigen or an epitope thereof, and/or an influenza nuclear protein antigen or an epitope thereof, stimulates or modulates an immunological response against influenza.

13. An adenoviral vector for use according to claim 12, wherein the response is induced by the vector expressing the nucleic acid in cells of the animal.

14. An adenoviral vector for use according to claim 12 or 13, wherein the animal is a vertebrate.

15. An adenoviral vector for use according to claim 14, wherein the vertebrate is a bird or a mammal.

16. An adenoviral vector for use according to claim 14, wherein the vertebrate is a human or a companion, a domesticated, a food-or-feed producing, a livestock, a game, a racing or a sport animal.

17. An adenoviral vector for use according to claim 15, wherein the animal is a cow, a horse, a dog, a cat, a goat, a sheep, a pig, or a chicken, a duck or a turkey.

18. An adenoviral vector for use according to any one of claims 11 to 17, wherein the adenoviral vector comprises a human adenovirus.

19. An adenoviral vector for use according to any one of claims 11 to 17, wherein the adenoviral vector comprises a canine adenovirus.

20. An adenoviral vector for use according to any one of claims 11 to 19, wherein the adenoviral vector comprises a viral vector having all viral genes deleted therefrom.

21. A non-invasive vaccine for mucosal administration comprising an adenoviral vector, wherein the vector contains and expresses an exogenous nucleic acid encoding an influenza hemagglutinin antigen or an epitope thereof, and/or an influenza nuclear protein antigen or an epitope thereof, and wherein the vector is deleted in its E1 and E3 regions for use in stimulating an immunological response against influenza in an animal, wherein the non-invasive vaccine for mucosal administration is to be delivered to the nasal mucosa.

22. A non-invasive vaccine for use according to claim 21, wherein the animal is a vertebrate.

23. A non-invasive vaccine for use according to claim 22, wherein the vertebrate is a bird or a mammal.

24. A non-invasive vaccine for use according to claim 22, wherein the vertebrate is a human or a companion, a domesticated, a food-or-feed producing, a livestock, a game, a racing or a sport animal.

25. A non-invasive vaccine for use according to claim 23, wherein the animal is a cow, a horse, a dog, a cat, a goat, a sheep, a pig, or a chicken, a duck or a turkey.

26. A non-invasive vaccine for use according to claim 21, wherein the adenoviral vector comprises a human adenovirus.

27. A non-invasive vaccine for use according to claim 21, wherein the adenoviral vector comprises a canine adenovirus.

28. A non-invasive vaccine for mucosal administration for use according to any one of claims 21 to 27, wherein the adenoviral vector comprises a viral vector having all viral genes deleted therefrom.

29. A kit comprising a non-invasive vaccine for use according to any one of claims 21 to 28 comprising in a first container the vector and in a second container a pharmaceutically acceptable carrier or diluent for administering the vector intranasally; wherein the containers are optionally present in the same package or are in separate packages; and the kit optionally contains instructions for admixture of the vector and the carrier or diluents and/or administration.

30. The kit according to claim 29, further comprising a delivery device in a third container; wherein the third container is optionally present in the same package as one or both of the first and second containers or is in a package separate from the first and second containers; and wherein the kit optionally contains instructions for installing the vector or vector and carrier or diluent into or onto the delivery device and/or for administration or application of the delivery device.

## Patentansprüche

1. Verwendung eines adenoviralen Vektors zur Herstellung eines nichtinvasiven Impfstoffes zur mukosalen Verabreichung, wobei der Vektor eine exogene Nukleinsäure enthält und exprimiert, die für ein Influenza Hämagglutininantigen oder ein Epitop davon und/oder für ein Influenza Nukleoproteinantigen oder ein Epitop davon kodiert, und der Vektor in seinen E1 und E3-Regionen deletiert ist und wobei der nichtinvasive Impfstoff zur mukosalen Verabreichung über die Nasenschleimhaut verabreicht wird.

2. Verwendung gemäß Anspruch 1, wobei die Expression eines Influenza Hämagglutininantigens oder eines Epitops davon und/oder eines Influenza Nukleoproteinantigens oder eines Epitops davon in einem Tier die Immunantwort gegen Influenza stimuliert oder moduliert.

3. Verwendung gemäß Anspruch 2, wobei die Antwort durch den Vektor, der die Nukleinsäure in Zellen des Tieres exprimiert, ausgelöst wird.

4. Verwendung gemäß Anspruch 2 oder 3, wobei das Tier ein Wirbeltier ist.

5. Verwendung gemäß Anspruch 4, wobei das Wirbeltier ein Vogel oder ein Säugetier ist.

6. Verwendung gemäß Anspruch 4, wobei das Wirbeltier ein Mensch oder ein Haustier, ein Nutztier, ein Nahrungs- oder Futtermittel produzierendes Tier, ein Vieh, ein Wild, oder ein Tier für Renn- oder Sportzwecke ist.

7. Verwendung gemäß Anspruch 5, wobei das Tier ein Rind, ein Pferd, ein Hund, eine Katze, eine Ziege, ein Schaf, ein Schwein oder ein Huhn, eine Ente oder ein Truthahn ist.

8. Verwendung gemäß einem der Ansprüche 1 bis 7, wobei der adenovirale Vektor ein humanes Adenovirus umfasst.

9. Verwendung gemäß einem der Ansprüche 1 bis 7, wobei der adenovirale Vektor ein Hunde-Adenovirus umfasst.

10. Verwendung gemäß einem der Ansprüche 1 bis 9, wobei der adenovirale Vektor einen viralen Vektor umfasst, von dem alle viralen Gene deletiert wurden.

11. Ein adenoviraler Vektor, der eine exogene Nukleinsäure enthält und exprimiert, die für ein Influenza Hämagglutininantigen oder ein Epitop davon und/oder für ein Influenza Nukleoproteinantigen oder ein Epitop davon kodiert, und welcher in seinen E1 und E3-Regionen deletiert ist, zur Verwendung als nichtinvasiven Impfstoff zur mukosalen Verabreichung, wobei der nichtinvasive Impfstoff zur mukosalen Verabreichung über die Nasenschleimhaut verabreicht wird.

12. Ein adenoviraler Vektor zur Verwendung gemäß Anspruch 11, wobei die Expression eines Influenza Hämagglutininantigens oder eines Epitops davon und/oder eines Influenza Nukleoproteinantigens oder eines Epitops davon in einem Tier die Immunantwort gegen Influenza stimuliert oder moduliert.

13. Ein adenoviraler Vektor zur Verwendung gemäß Anspruch 12, wobei die Antwort durch den Vektor, der die Nukleinsäure in Zellen des Tieres exprimiert, ausgelöst wird.

14. Ein adenoviraler Vektor zur Verwendung gemäß Anspruch 12 oder 13, wobei das Tier ein Wirbeltier ist.

15. Ein adenoviraler Vektor zur Verwendung gemäß Anspruch 14, wobei das Wirbeltier ein Vogel oder ein Säugetier ist.

16. Ein adenoviraler Vektor zur Verwendung gemäß Anspruch 14, wobei das Wirbeltier ein Mensch oder ein Haustier, ein Nutztier, ein Nahrungs- oder Futtermittel produzierendes Tier, ein Vieh, ein Wild, oder ein Tier für Renn- oder Sportzwecke ist.

17. Ein adenoviraler Vektor zur Verwendung gemäß Anspruch 15, wobei das Tier ein Rind, ein Pferd, ein Hund, eine Katze, eine Ziege, ein Schaf, ein Schwein oder ein Huhn, eine Ente oder ein Truthahn ist.

18. Ein adenoviraler Vektor zur Verwendung gemäß einem der Ansprüche 11 bis 17, wobei der adenovirale Vektor ein humanes Adenovirus umfasst.

19. Ein adenoviraler Vektor zur Verwendung gemäß einem der Ansprüche 11 bis 17, wobei der adenovirale Vektor ein Hunde-Adenovirus umfasst.

20. Ein adenoviraler Vektor zur Verwendung gemäß einem der Ansprüche 11 bis 19, wobei der adenovirale Vektor einen viralen Vektor umfasst, von dem alle viralen Gene deletiert wurden.

21. Ein nichtinvasiver Impfstoff zur mukosalen Verabreichung umfassend einen adenoviralen Vektor, wobei der Vektor eine exogene Nukleinsäure enthält und exprimiert, die für ein Influenza Hämagglutininantigen oder ein Epitop davon und/oder für ein Influenza Nukleoproteinantigen oder ein Epitop davon kodiert, und wobei der Vektor in seinen E1 und E3-Regionen deletiert ist, zur Verwendung bei der Stimulierung einer Immunantwort gegen Influenza in einem Tier, wobei der nichtinvasive Impfstoff zur mukosalen Verabreichung über die Nasenschleimhaut verabreicht wird.

22. Ein nichtinvasiver Impfstoff zur Verwendung gemäß Anspruch 21, wobei das Tier ein Wirbeltier ist.

23. Ein nichtinvasiver Impfstoff zur Verwendung gemäß Anspruch 22, wobei das Wirbeltier ein Vogel oder ein Säugetier ist.

24. Ein nichtinvasiver Impfstoff zur Verwendung gemäß Anspruch 22, wobei das Wirbeltier ein Mensch oder ein Haustier, ein Nutztier, ein Nahrungs- oder Futtermittel produzierendes Tier, ein Vieh, ein Wild, oder ein Tier für Renn- oder Sportzwecke ist.

25. Ein nichtinvasiver Impfstoff zur Verwendung gemäß Anspruch 23, wobei das Tier ein Rind, ein Pferd, ein Hund, eine Katze, eine Ziege, ein Schaf, ein Schwein oder ein Huhn, eine Ente oder ein Truthahn ist.

26. Ein nichtinvasiver Impfstoff zur Verwendung gemäß Anspruch 21, wobei der adenovirale Vektor ein humanes Adenovirus umfasst.

27. Ein nichtinvasiver Impfstoff zur Verwendung gemäß Anspruch 21, wobei der adenovirale Vektor ein Hunde-Adenovirus umfasst.

28. Ein nichtinvasiver Impfstoff zur mukosalen Verabreichung zur Verwendung gemäß einem der Ansprüche 21 bis 27, wobei der adenovirale Vektor einen viralen Vektor umfasst, von dem alle viralen Gene deletiert wurden.

29. Ein Kit umfassend einen nichtinvasiven Impfstoff zur Verwendung gemäß einem der Ansprüche 21 bis 28, umfassend in einem ersten Behälter den Vektor und in einem zweiten Behälter eine pharmazeutisch verträgliche Trägersubstanz oder ein Verdünnungsmittel zur intranasalen Verabreichung des Vektors; wobei sich die Behälter wahlweise in derselben Verpackung oder in separaten Verpackungen befinden; und der Kit wahlweise eine Anleitung zum Vermischen des Vektors und der Trägersubstanz oder des Verdünnungsmittels und/oder zur Verabreichung enthält.

30. Das Kit gemäß Anspruch 29, ferner umfassend eine Verabreichungsvorrichtung in eine dritten Behälter; wobei sich der dritte Behälter wahlweise in derselben Verpackung wie einer oder beide der ersten und zweiten Behälter befindet oder in Verpackung getrennt von den ersten und zweiten Behältern; und wobei der Kit wahlweise eine Anleitung zum Installieren des Vektors oder des Vektors und der Trägersubstanz oder des Verdünnungsmittels in oder auf die Verabreichungsvorrichtung und/oder zur Verabreichung oder Anwendung der Verabreichungsvorrichtung enthält.

## Revendications

1. Utilisation d'un vecteur adénoviral pour la préparation d'un vaccin non invasif pour l'administration aux muqueuses, dans laquelle le vecteur contient et exprime un acide nucléique exogène codant pour un antigène hémagglutinine grippal ou un de ses épitopes, et/ou un antigène protéique nucléaire grippal ou un de ses épitopes, et le vecteur présente une délétion dans ses régions E1 et E3, et dans laquelle le vaccin non invasif pour l'administration aux muqueuses doit être délivré à la muqueuse nasale.

2. Utilisation suivant la revendication 1, dans laquelle l'expression chez un animal d'un antigène hémagglutinine grippal ou d'un de ses épitopes, et/ou d'un antigène protéique nucléaire grippal ou d'un de ses épitopes, stimule ou module une réponse immunologique contre la grippe.

3. Utilisation suivant la revendication 2, dans laquelle la réponse est induite par le vecteur exprimant l'acide nucléique dans des cellules de l'animal.

4. Utilisation suivant la revendication 2 ou 3, dans laquelle l'animal est un vertébré.

5. Utilisation suivant la revendication 4, dans laquelle le vertébré est un oiseau ou un mammifère.

6. Utilisation suivant la revendication 4, dans laquelle le vertébré est un être humain ou un animal de compagnie, un animal domestique, un animal producteur de nourriture humaine ou animale, un animal servant de bétail, un animal servant de gibier, un animal de course ou un animal de sport.

7. Utilisation suivant la revendication 5, dans laquelle l'animal est une vache, un cheval, un chien, un chat, une chèvre, un mouton, un porc ou un poulet, un canard ou une dinde.

8. Utilisation suivant l'une quelconque des revendications 1 à 7, dans laquelle le vecteur adénoviral comprend un adénovirus humain.

9. Utilisation suivant l'une quelconque des revendications 1 à 7, dans laquelle le vecteur adénoviral comprend un adénovirus canin.

10. Utilisation suivant l'une quelconque des revendications 1 à 9, dans laquelle le vecteur adénoviral comprend un vecteur viral présentant une délétion de tous les gènes viraux.

11. Vecteur adénoviral qui contient et exprime un acide nucléique exogène codant pour un antigène hémagglutinine grippal ou un de ses épitopes, et/ou un antigène protéique nucléaire grippal ou un de ses épitopes, et qui présente une délétion dans ses régions E1 et E3, pour une utilisation comme vaccin non invasif pour l'administration aux muqueuses, ledit vaccin non invasif pour l'administration aux muqueuses étant destiné à être délivré à la muqueuse nasale.

12. Vecteur adénoviral pour une utilisation suivant la revendication 11, l'expression chez un animal d'un antigène hémagglutinine grippal ou d'un de ses épitopes, et/ou d'un antigène protéique nucléaire grippal ou d'un de ses épitopes stimulant ou modulant une réponse immunologique contre la grippe.

13. Vecteur adénoviral pour une utilisation suivant la revendication 12, la réponse étant induite par le vecteur exprimant l'acide nucléique dans des cellules de l'animal.

14. Vecteur adénoviral pour une utilisation suivant la revendication 12 ou 13, l'animal étant un vertébré.

15. Vecteur adénoviral pour une utilisation suivant la revendication 14, le vertébré étant un oiseau ou un mammifère.

16. Vecteur adénoviral pour une utilisation suivant la revendication 14, le vertébré étant un être humain ou un animal de compagnie, un animal domestique, un animal producteur de nourriture pour l'homme ou les animaux, un animal servant de bétail, un animal servant de gibier, un animal de course ou un animal de sport.

17. Vecteur adénoviral pour une utilisation suivant la revendication 15, l'animal étant une vache, un cheval, un chien, un chat, une chèvre, un mouton, un porc ou un poulet, un canard ou une dinde.

18. Vecteur adénoviral pour une utilisation suivant l'une quelconque des revendications 11 à 17, ledit vecteur adénoviral comprenant un adénovirus humain.

19. Vecteur adénoviral pour une utilisation suivant l'une quelconque des revendications 11 à 17, ledit vecteur adénoviral comprenant un adénovirus canin.

20. Vecteur adénoviral pour une utilisation suivant l'une quelconque des revendications 11 à 19, ledit vecteur adénoviral comprenant un vecteur viral présentant une délétion de tous les gènes viraux.

21. Vaccin non invasif pour l'administration aux muqueuses, comprenant un vecteur adénoviral, dans lequel le vecteur contient et exprime un acide nucléique exogène codant pour un antigène hémagglutinine grippal ou un de ses épitopes, et/ou un antigène protéique nucléaire grippal ou un de ses épitopes, et dans lequel le vecteur présente une délétion dans ses régions E1 et E3, pour une utilisation dans la stimulation d'une réponse immunologique contre la grippe chez un animal, ledit vaccin non invasif pour l'administration aux muqueuses étant destiné à être délivré à la muqueuse nasale.

22. Vaccin non invasif pour une utilisation suivant la revendication 21, l'animal étant un vertébré.

23. Vaccin non invasif pour une utilisation suivant la revendication 22, le vertébré étant un oiseau ou un mammifère.

24. Vaccin non invasif pour une utilisation suivant la revendication 22, le vertébré étant un être humain ou un animal de compagnie, un animal domestique, un animal producteur de nourriture pour l'homme ou les animaux, un animal servant de bétail, un animal servant de gibier, un animal de course ou un animal de sport.

25. Vaccin non invasif pour une utilisation suivant la revendication 23, l'animal étant une vache, un cheval, un chien, un chat, une chèvre, un mouton, un porc ou un poulet, un canard ou une dinde.

26. Vaccin non invasif pour une utilisation suivant la revendication 21, dans lequel le vecteur adénoviral comprend un adénovirus humain.

27. Vaccin non invasif pour une utilisation suivant la revendication 21, dans lequel le vecteur adénoviral comprend un adénovirus canin.

28. Vaccin non invasif pour l'administration aux muqueuses, pour une utilisation suivant l'une quelconque des revendications 21 à 27, dans lequel le vecteur adénoviral comprend un vecteur viral présentant une délétion de tous les gènes viraux.

29. Kit comprenant un vaccin non invasif pour une utilisation suivant l'une quelconque des revendications 21 à 28, comprenant, dans un premier récipient, le vecteur et, dans un second récipient, un support ou diluant pharmaceutiquement acceptable pour l'administration du vecteur par voie intranasale ; dans lequel les récipients sont éventuellement présents dans le même emballage ou sont présents dans des emballages séparés ; ledit kit contenant éventuellement des instructions pour le mélange du vecteur et du support ou diluant et/ou pour l'administration.

30. Kit suivant la revendication 29, comprenant en outre un dispositif d'administration dans un troisième récipient ; dans lequel le troisième récipient est éventuellement présent dans le même emballage que l'un des ou les deux premier et second récipients ou bien est présent dans un emballage séparé des premier et second récipients ; ledit kit contenant éventuellement des instructions pour l'installation du vecteur ou du vecteur et du support ou diluant dans ou sur le dispositif d'administration et/ou pour l'administration ou l'application du dispositif d'administration.
